# EUROPEAN PATENT SPECIFICATION

(11) **EP 1 618 208 B1**
(45) Date of publication and mention of the grant of the patent: **28.04.2010**
(21) Application number: 04721788.0
(22) Date of filing: 19.03.2004
(51) Int. Cl.: C12Q 1/68

(54) **PLASMINOGEN ACTIVATOR INHIBITOR-1 (PAI-1) HAPLOTYPES USEFUL AS INDICATORS OF PATIENT OUTCOME**
ALS INDIKATOREN ZUR PATIENTENPROGNOSE GEEIGNETE HAPLOTYPEN VON PLASMINOGENAKTIVATOR-INHIBITOR-1 (PAI-1)
HAPLOTYPES DE L'INHIBITEUR 1 D'ACTIVATEUR DE PLASMINOGENE (PAI-1) UTILES COMME INDICATEURS D'EVOLUTION CHEZ DES PATIENTS

(30) Priority: 19.03.2003 US 455550 P
(43) Date of publication of application: 25.01.2006
(60) Divisional application: 10154345.2
(73) Proprietor: THE UNIVERSITY OF BRITISH COLUMBIA, Vancouver, British Columbia V6T 1Z3 (CA)
(72) Inventor: RUSSELL, James, A., Vancouver, British Columbia V6M 1H8 (CA); WALLEY, Keith, R., North Vancouver, British Columbia V7N 4M9 (CA)
(74) Representative: HOFFMANN EITLE
(86) International application number: PCT/CA2004/000424
(87) International publication number: WO 2004/083457

(56) References cited:
- WO-A1-98/53098
- US-A1- 2002 086 838
- BENZA: "Association of a Gene Polymorphism for PAI-1 with Primary Pulmonary Hypertension" CARDIOPULMONARY CRITICAL CARE NEWSLETTER, [Online] XP002287063 Retrieved from the Internet: URL:http://www.americanheart.org/downloada ble/heart/1017868019324CPCCSprng02.pdf> [retrieved on 2002-03-01]
- HERMANS PETER W M ET AL: "4G/5G promoter polymorphism in the plasminogen-activator-inhibitor-1 gene and outcome of meningococcal disease" LANCET (NORTH AMERICAN EDITION), vol. 354, no. 9178, 14 August 1999 (1999-08-14), pages 556-560, XP002287064 ISSN: 0099-5355
- CATCHPOOLE D R ET AL: "The potential tumour suppressor role for caspase-9 (CASP9) in the childhood malignancy, neuroblastoma" EUROPEAN JOURNAL OF CANCER, PERGAMON PRESS, OXFORD, GB, vol. 37, no. 17, 2001, pages 2217-2221, XP004309829 ISSN: 0959-8049
- NOGUCHI E ET AL: "IDENTIFICATION OF MISSENSE MUTATION IN THE IL12B GENE: LACK OF ASSOCIATION BETWEEN IL12B POLYMORPHISMS AND ASTHMA AND ALLERGIC RHINITIS IN THE JAPANESE POPULATION" GENES AND IMMUNITY, NATURE PUBLISHING GROUP, GB, vol. 2, 2001, pages 401-403, XP002908314 ISSN: 1466-4879
- GAILLARD M-C ET AL: "Identification of DNA polymorphisms associated with the V type alpha1-antitrypsin gene" BIOCHIMICA ET BIOPHYSICA ACTA . GENE STRUCTURE AND EXPRESSION, ELSEVIER, AMSTERDAM, NL, vol. 1444, no. 2, 16 February 1999 (1999-02-16), pages 166-170, XP004275307 ISSN: 0167-4781
- DAWSON SALLY J ET AL: "The two allele sequences of a common polymorphism in the promoter of the plasminogen activator inhibitor-1 (PAI-1) gene respond differently to interleukin-1 in HepG2 cells" JOURNAL OF BIOLOGICAL CHEMISTRY, vol. 268, no. 15, 1993, pages 10739-10745, XP002287069 ISSN: 0021-9258

## Description

### RELATED APPLICATION DATA

This application relates to U.S. provisional application No. 60/455,550 filed March 19, 2003.

### FIELD OF THE INVENTION

The present invention relates to the field of assessment and treatment of patients and more specifically, to the determination of a patients genotype for one or more polymorphisms to determine the patient's prognosis and to determine therapy.

### BACKGROUND OF THE INVENTION

Systemic inflammatory response syndrome (SIRS) is characterized by increased inflammation (anti-inflammatory processes), increased coagulation (anti-coagulant processes), and decreased fibrinolysis (SUFFREDINI AF et al. New England Journal of Medicine (1989) 320(18):1165-72; HESSELVIK JF et al. Critical Care Medicine (1989) 17(8):724-33; PHILIPPE J et al. Thrombosis & Haemostasis (1991) 65(3):291-5; LORENTE JA et al. Chest (1993) 103(5):1536-42; FOURRIER F et al. Chest (1992) 101(3):816-23; MESTERS RM et al. (1996) 88(3):881.6.). Septic and non-septic stimuli such as bacterial endotoxin and cardiopulmonary bypass (CPB), are implicated in the activation of the coagulation system and trigger a systemic inflammatory response syndrome (SIRS). Plasminogen-activator-inhibitor-1 (PAI-1) is associated with decreased fibrinolysis (BINDER BR et al. News in Physiological Sciences (2002) 17:56-61). PAI-1 levels are reported increased in patients with sepsis (MESTERS RM et al. Thrombosis & Haemostasis. (1996) 75(6):902-7; PRALONG G et al. Thrombosis & Haemostasis. (1989) 61(3):459-62), in meningococcemia (HERMANS PW et al. Lancet. (1999) 354(9178):556-60), and in trauma (MENGES T et al. Lancet (2001) 357(9262):1096-7). Furthermore, increased PAI-1 levels are associated with decreased survival and increased organ dysfunction (MESTERS RM et al. (1996); PRALONG G et al. (1989)).

The human PAI-1. gene maps to chromosome 7 q21-q22 and extends over about 12kb. A representative *Homo sapiens* PAI-1 gene sequence is listed in GenBank under accession number AF386492 (shown herein as SEQ ID NO.: 1). DAWSON et al. (Journal of Biological Chemistry (1993) 268(15):10739-45) identified an insertion/deletion polymorphism (4G/5G) at position - 675 of the PAI-1 promoter sequence, which corresponds to position 837 of SEQ ID NO.: 1. The 4G allele is a single base pair deletion promoter polymorphism of the PAI-1 gene and is associated with increased protein levels of PAI-1 (DAWSON SJ et al. (1993); DAWSON SJ et al. Arteriosclerosis & Thrombosis (1991) 11(1):183-90). The 4G allele of this single nucleotide polymorphism (SNP) is associated with increased risk of deep venous thrombosis (SEGUI R et al. British Journal of Haematology (2000) 111(1):122-8), stroke (HINDORFF LA et al. Journal of Cardiovascular Risk (2002) 9(2):131-7), acute myocardial infarction (BOEKHOLDT SM et al. Circulation (2001) 104(25):3063-8; ERIKSSON P et al. PNAS (1995) 92(6):1851-5.), late lumen loss after coronary artery stent placement (ORTLEPPG JR et al. Clinical Cardiology (2001) 24(9):585-91), and sudden cardiac death (ANVARI A et al. Thrombosis Research (2001) 103(2):103-7; MIKKELSSON J et al. Thrombosis & Haemostasis (2000) 84(1):78-82). In the critically ill, the 4G allele is also associated with decreased survival in patients who have had severe trauma (MENGES T et al. Lancet (2001) 357(9262):1096-7) and patients who had meningococcemia (HERMANS PW et al. Lancet. (1999) 354(9178):556-60) as well as increased risk of shock in patients who had meningococcemia (WESTENDORP RG et al. Lancet (1999) 354(9178):561-3). The PAI-1 4G genotype has also been associated with adverse patient outcomes ((MENGES et al. (2001); HERMANS et al. (1999); WESTENDORP RG et al. (1999); ENDLER G et al. British Journal of Haematology (2000) 110(2):469-71; GARDEMANN A et al. Thrombosis & Haemostasis (1999) 82(3):1121-6; HOOPER WC et al. Thrombosis Research (2000) 99(3):223-30; JONES K et al. European Journal of Vascular & Endovascular Surgery (2002) 23(5):421-5; HARALAMBOUS E. et al. Crit Care Med (2003) 31(12):2788-93; and ROEST M et al. Circulation (2000) 101(1):67-70). The 4G/4G genotype of PAI-1 was associated with PAI-1 levels in patients suffering from acute lung injury (RUSSELL JA Crit Care Med. (2003) 31 (4):S243-S247).

Furthermore a polymorphism at position 14,699 of the PAI-1 gene has been linked to primary pulmonary hypertension (BENZA et al.: "Association of a Gene Polymorphism for PAI-1 with Primary Pulmonary Hypertension", Cardiopulmonary Critical Care Newsletter).

### SUMMARY OF THE INVENTION

This invention is based in part on the surprising discovery that the Plasminogen-Activator-Inhibitor-1 (PAI-1) 4G/5G promoter polymorphism is not as accurate a predictor of patient outcome as the SNP at position 12580 of SEQ ID NO:1. Furthermore, various other PAI-1 polymorphisms in total linkage disequilibrium (LD) with SNP 12580 are identified as being useful for patient screening, as an indication of patient outcome, or for prognosis for recovery from an inflammatory condition or to tailor therapy for patients with inflammatory conditions or for the selection of therpaies for patients based on their PAI-1 12580 genotype and hapoltype. Surprisingly, the SNPs identified herein as predictors of patient outcome also correspond with lower PAI-1 levels.

In accordance with one aspect of the invention, methods are provided for obtaining a prognosis for a patient having or at risk of developing an inflammatory condition, the method comprising determining a genotype including one or more polymorphism sites in the PAI-1 gene for the patient, wherein said genotype is indicative of an ability of the patient to recover from an inflammatory condition and provided the genotype selected does not correspond to only the 837 position of SEQ ID NO.: 1.

In accordance with another aspect of the invention, methods are provided for obtaining a prognosis for a patient having or at risk of developing an inflammatory condition, the method comprising determining a genotype of one or more polymorphism sites in the PAI-1 gene for the patient selected from the polymorphism sites corresponding to positions 664, 2037, 2362, 2852, 5834, 5878, 7343, 13605, 7365, 7729, 7771, 12750, 4588, 5404, 5686, 5984, 11312, 2846, 6821, 9759, 10381, 7365, 7729, 7771, 12750, 4588, 5404, 5686, 5984, 11312, 5645, 7121, 7437, 8070, 8406, 9463, 9466, 12219, 12580, 13889, 14440, wherein said genotype is indicative of an ability of the patient to recover from an inflammatory condition.

In accordance with one aspect of the invention, methods are provided for obtaining a prognosis for a patient having or at risk of developing an inflammatory condition, the method comprising determining a genotype including one or more polymorphism sites in the PAI-1 gene for the patient, wherein said genotype is indicative of an ability of the patient to recover from an inflammatory condition and the polymorphism is selected from the region defined by positions 1581 to 14544 of SEQ ID NO.: 1.

The polymorphism site may correspond to position 12580 of SEQ ID NO.: 1 or a polymorphism site linked thereto. Alternatively, the polymorphism site may correspond to positions 5645, 7121, 7437, 8070, 8406, 9463, 9466, 12219, 13889 or 14440 in SEQ ID NO: 1.

Genotype may also be determined at a combination of multiple polymorphism sites, the combination being selected from the group of positions corresponds to SEQ ID NO: consisting of:
664 and 2037;
664 and 2362;
664 and 2852;
664 and 5834;
837 and 2037;
837 and 2362;
837 and 2852;
837 and 5834;

| | | | | | |
|---|---|---|---|---|---|
| one of | 5878 | and one of | 7365 | and one of | 4588 |
| | 7343 | | 7729 | | 5404 |
| | 13605 | | 7771 | | 5686 |
| | | | 12750 | | 5984 |
| | | | | | 11312; |

and

| | | | | | | | |
|---|---|---|---|---|---|---|---|
| one of | 2846 | and | 10381 | and one of | 7365 | and one of | 4588 |
| | 6821 | | | | 7729 | | 5404 |
| | 9759 | | | | 7771 | | 5686 |
| | | | | | 12750 | | 5984 |
| | | | | | | | 11312. |

Alternatively, genotypes may be determined for polymorphism located within the 1581-14544 of SEQ ID NO:1, to exclude polymorphisms within the promoter region. Preferably, genotypes would be determined for polymorphisms selected from the group consisting of 5645, 7121, 7437, 8070, 8406, 9463, 9466, 12219, 12580, 13889, or 14440 or SEQ ID NO:1.

In accordance with another aspect of the invention, methods are provided for further comparing the genotype so determined with known genotypes, which are indicative of a prognosis for recovery from the same inflammatory condition as for the patient or another inflammatory condition.

The genotype of the patient may be indicative of a decreased likelihood of recovery from an inflammatory condition or indicative of a prognosis of severe cardiovascular or respiratory dysfunction in critically ill patients. Furthermore, such a genotype may be selected from the group of single polymorphism sites and combined polymorphism sites consisting of:
5645 T;
7121 G;
7437 T;
8070 A;
8406 C;
9463 G;
9466 T;
12219 C;
12580 G;
13889 C;
14440 A;
664 A and 2037 T;
664 A and 2362 -;
664 A and 2852 A;
664 A and 5834 A;
837 - and 2037 T;
837-and2362-;
837-and 2852 A; and
837 - and 5834 A.

The genotype of the patient may be indicative of an increased likelihood of recovery from an inflammatory condition or indicative of a prognosis of less severe cardiovascular or respiratory dysfunction in critically ill patients. Furthermore, such a genotype may be selected from the group of single polymorphism sites and combined polymorphism sites consisting of:
5645 C;
7121 A;
7437 C;
8070 G;
8406 T;
9463 A;
9466 C;
12219 T;
12580 T;
13889 T;
14440 G;

| | | | | | |
|---|---|---|---|---|---|
| one of | 5878 G | and one of | 7365 T | and one of | 4588 T |
| | 7343 G | | 7729 + | | 5404 G |
| | 13605 A | | 7771 A | | 5686 A |
| | | | 12750 A | | 5984 A |
| | | | | | 11312 A; |

and

| | | | | | | |
|---|---|---|---|---|---|---|
| one of | 2846 A | and 10381 T | and one of | 7365 T | and one of | 4588 T |
| | 6821 T | | | 7729 + | | 5404 G |
| | 9759 G | | | 7771 A | | 5686 A |
| | | | | 12750 A | | 5984 A |
| | | | | | | 11312 A. |

In accordance with another aspect of the invention, methods are provided for identifying a polymorphism in a PAI-1 gene sequence that correlates with patient prognosis. Where the method comprises obtaining PAI-1 gene sequence information from a group of patients, identifying a site of at least one polymorphism in the PAI-1 gene, determining genotypes at the site for individual patients in the group, determining an ability of individual patients in the group to recover from the inflammatory condition and correlating genotypes determined with patient with potential therapies.

The correlation procedure may be repeated on a patient population of sufficient size to achieve a statistically significant correlation.

The methods may further comprise steps of obtaining PAI-1 gene sequence of the patient or obtaining a nucleic acid sample from the patient. The determining of genotype may be performed on a nucleic acid sample from the patient.

Where the genotype of the patient corresponding to the nucleotide in position 12580, is G, the prognosis maybe indicative of a decreased likelihood of recovery from an inflammatory condition or of severe cardiovascular or respiratory dysfunction in critically ill patients.

Where the genotype of the patient corresponding to the nucleotide in position 12580, is T, the prognosis may be indicative of a increased likelihood of recovery from an inflammatory condition or of less severe cardiovascular or respiratory dysfunction in critically ill patients.

The inflammatory condition may be selected from the group consisting of: sepsis, septicemia, pneumonia, septic shock, systemic inflammatory response syndrome (SIRS), Acute Respiratory Distress Syndrome (ARDS), acute lung injury, infection, pancreatitis, bacteremia, peritonitis, abdominal abscess, inflammation due to trauma, inflammation due to surgery, chronic inflammatory disease, ischemia, ischemia-reperfusion injury of an organ or tissue, tissue damage due to disease, tissue damage due to chemotherapy or radiotherapy, and reactions to ingested, inhaled, infused, injected, or delivered substances, glomerulonephritis, bowel infection, opportunistic infections, and for patients undergoing major surgery or dialysis, patients who are immunocompromised, patients on immunosuppressive agents, patients with HIV/AIDS, patients with suspected endocarditis, patients with fever, patients with fever of unknown origin, patients with cystic fibrosis, patients with diabetes mellitus, patients with chronic renal failure, patients with bronchiectasis, patients with chronic obstructive lung disease, chronic bronchitis, emphysema, or asthma, patients with febrile neutropenia, patients with meningitis, patients with septic arthritis, patients with urinary tract infection, patients with necrotizing fasciitis, patients with other suspected Group A streptococcus infection, patients who have had a splenectomy, patients with recurrent or suspected enterococcus infection, other medical and surgical conditions associated with increased risk of infection, Gram positive sepsis, Gram negative sepsis, culture negative sepsis, fungal sepsis, meningococcemia, post-pump syndrome, cardiac stun syndrome, myocardial infarction, stroke, congestive heart failure, hepatitis, epiglotittis, E. coli 0157:H7, malaria, gas gangrene, toxic shock syndrome, mycobacterial tuberculosis, Pneumocystic carinii, pneumonia, Leishmaniasis, hemolytic uremic syndrome/thrombotic thrombocytopenic purpura, Dengue hemorrhaigic fever, pelvic inflammatory disease, Legionella, Lyme disease, Influenza A, Epstein-Barr virus, encephalitis, inflammatory diseases and autoimmunity including Rheumatoid arthritis, osteoarthritis, systemic lupus erythematosus, inflammatory bowel disease, idiopathic pulmonary fibrosis, sarcoidosis, hypersensitivity pneumonitis, systemic vasculitis, Wegener's granulomatosis, transplants including heart, liver, lung kidney bone marrow, graft-versus-host disease, transplant rejection, sickle cell anemia, nephrotic syndrome, toxicity of agents such as OKT3, cytokine therapy, and cirrhosis.

The determining of a genotype may comprise one or more of: restriction fragment length analysis; sequencing; hybridization; oligonucleotide ligation assay; ligation rolling circle amplification; 5' nuclease assay; polymerase proofreading methods; allele specific PCR; and reading sequence data.

In accordance with another aspect of the invention, there is provided a kit for determining a genotype at a defined nucleotide position within a polymorphism site in a PAI-1 gene sequence from a patient to provide a prognosis of the patient's ability to recover from an inflammatory condition, the kit comprising, in a package a restriction enzyme capable of distinguishing alternate nucleotides at the polymorphism site or a labeled oligonucleotide having sufficient complementary to the polymorphism site and capable of distinguishing said alternate nucleotides:

The alternate nucleotides may correspond to position 12580 of SEQ ID NO: 1, position 10 of SEQ ID NO: 2 or to a polymorphism linked thereto. The alternate nucleotides may also correspond to one or more of positions 5645, 7121, 7437, 8070, 8406, 9463, 9466, 12219, 13889 or 14440 of SEQ ID NO: 1.

The kit comprising a restriction enzyme may also comprise an oligonucleotide or a set of oligonucleotides suitable to amplify a region surrounding the polymorphism site, a polymerization agent and instructions for using the kit to determine genotype.

In accordance with another aspect of the invention, methods are provided for determining patient prognosis in a patient having or at risk of developing an inflammatory condition, the method comprising detecting the identity of one or more polymorphisms in the PAI-1 promoter region, wherein the identity of said one or more polymorphisms is indicative of the ability of the patient to recover from the inflammatory condition.

In accordance with another aspect of the invention, methods are provided for patient screening, comprising the steps of (a) obtaining PAI-1 gene sequence information from a patient, and (b) determining the identity of one or more polymorphisms in the promoter region, wherein the one or more polymorphism may be indicative of the ability of a patient to recover from an inflammatory condition.

In accordance with another aspect of the invention methods are provided for patient screening whereby the method includes the steps of (a) selecting a patient based on risk of developing an inflammatory condition or having an inflammatory condition, (b) obtaining PAI-1 gene sequence information from the patient and (c) detecting the identity of one or more polymorphisms in the PAI-1 gene, wherein the polymorphism is indicative of the ability of a patient to recover from an inflammatory condition.

In accordance with another aspect of the invention methods are provided for selecting a group of patients to determine the efficacy of a candidate drug known or suspected of being useful for the treatment of an inflammatory condition, the method including determining a genotype for one or more polymorphism sites in the plasminogen-activator-inhibitor-1 gene for each patient, wherein said genotype is indicative of the patient's ability to recover from the inflammatory condition and sorting patients based on their genotype, provided that the polymorphism site is not solely a polymorphism at a site corresponding to position 837 of SEQ ID NO.: 1.

The method may include the additional step of comparing patient response to the candidate drug based on genotype of the patient. Response to the candidate drug may be decided by determining each patient's ability to recover from the inflammatory condition.

The above sequence positions refer to the sense strand of the PAI-1 gene. It will be obvious to a person skilled in the art that analysis could be conducted on the anti-sense strand to determine patient outcome.

More severe patient outcome or prognosis may be correlated with lower PAI-1 expression or conversely an indication of less severe patient outcome or prognosis may be correlated with higher PAI-1 expression, which is the opposite of what would be expected.

### BRIEF DESCRIPTION OF THE DRAWINGS

- Figure 1.: shows a haplotype map of plasminogen activator inhibitor-1 (PAI-1) in Caucasians, where the columns indicate polymorphic loci and rows indicate distinct haplotypes.
Light cells represent rare alleles, and dark cells represent common alleles.
Polymorphisms that provide the same information as the 12580G allele were chosen based on this haplotype map. These polymorphisms provide the same information as the 12580G allele.
- Figure 2.: shows the PAI-1 haplotype clades in an unrooted phylogenetic tree indicating the positions of haplotypes from Figure 1. Figure 2 was drawn using MEGA II where line length reflects relative evolutionary distance and each clade is circle.

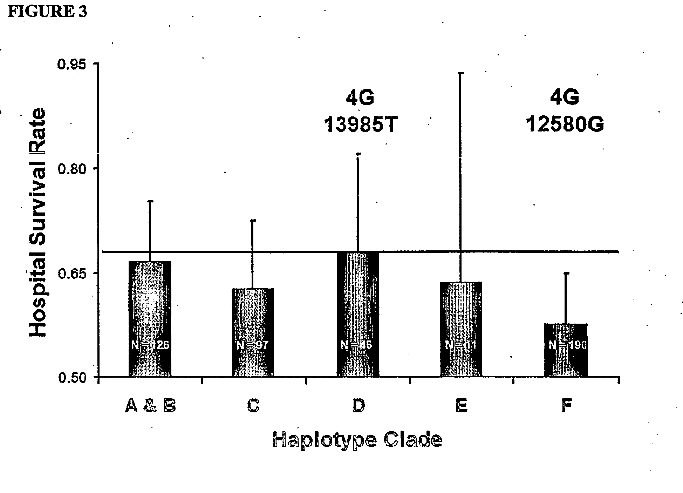

- Figure 3.: shows hospital survival for each of the haplotype clades of the PAI-1 gene. Error bars indicate 95% confidence intervals. Haplotypes containing the 4G allele are made up of 2 separate haplotype clades defined by: the 13985 T allele and the 12580 G allele. The group mean, excluding the 4G haplotype clade containing the 12580 G allele, is illustrated by the horizontal line. The mean and 95% confidence interval for the haplotype clade defined by the 12580 G allele falls below the mean of the rest of the group. In contrast, the 4G haplotype clade containing the 13985 T allele has the highest hospital survival of any haplotype. Data from patients were included twice, once for each haplotype that they possessed (homozygotes for a haplotype were included twice in that haplotype), so that the number of haplotypes (numbers within the data bars) add up to 2 times the total number of patients (235).
- Figure 4.: shows Days Alive and Free (DAF) of organ system failure over the 28 day study period by geneotype for the PAI-1 12580 allele.

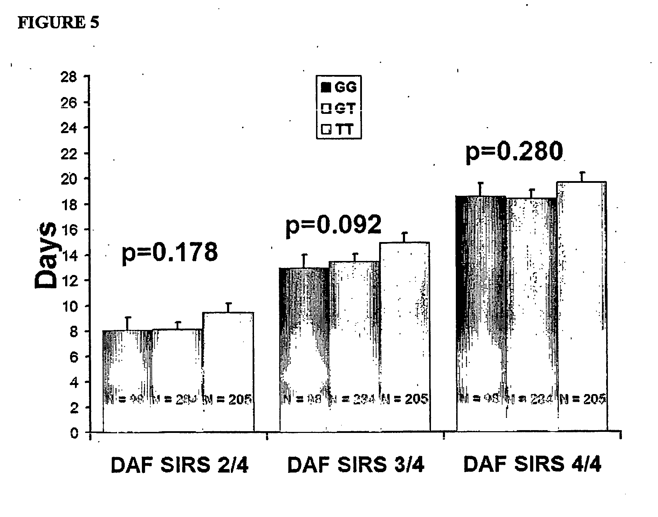

- Figure 5: shows days alive and free of fulfillment of 2, 3 or 4 out of 4 (2/4, 3/4 or 4/4) systemic inflammatory response syndrome (SIRS) criteria by PAI-1 12580 geneotype. The SIRS criteria are: 1) tachycardia (heart rate >100 bpm in the absence of beta blockers), 2) tachypnea (respiration rate > 20 breaths/min) or mechanically ventilated, 3) fever (temperature > 38°C) or hypothermia (temperature < 35.5°C), and 4) leukocytosis (total leukocyte count > 11,000/µL), using the guidelines established in the American College of Chest Physicians/Society of Critical Care Medicine Consensus Conference: definitions for sepsis and organ failure and guidelines for the use of innovative therapies in sepsis (Critical Care Medicine. (1992) 20(6):864-74). Each day that a patient was in the ICU the presence or absence of each SIRS criteria was scored, such that the total number of SIRS criteria fulfilled on each day in the ICU was tallied. Since fulfillment of SIRS criteria indicates poor outcome, more days alive and free of fulfillment of SIRS criteria indicates a better outcome.
- Figure 6.: shows the occurrence of Sepsis, and Septic Shock by geneotype for the PAI-1 12580 allele.
- Figure 7A.: shows days alive and free of respiratory dysfunction and mechanical ventilation by PAI-1 12580 genotype (GG, GT and TT separately) in 587 critically ill Caucasians with the systemic inflammatory response syndrome (SIRS).
- Figure 7B.: shows days alive and free of respiratory dysfunction and mechanical ventilation by PAI-1 12580 genotype (GG/GT vs TT) in 587 critically ill Caucasians with SIRS.
- Figure 8A.: shows days alive and free of respiratory dysfunction and mechanical ventilation by PAI-1 4G/5G (837) genotype (4G/4G, 4G/5G and 5G/5G separately) in 314 critically ill Caucasians with the systemic inflammatory response syndrome (SIRS).
- Figure 8B.: shows days alive and free of respiratory dysfunction and mechanical ventilation by PAI-1 4G/5G (837) allele (any 4G vs 5G/5G) in 314 critically ill Caucasians with SIRS.
- Figure 9A.: shows days alive and free of acute lung injury (ALI) and PₐO₂/FᵢO₂ (P/F) ratio greater than 300 mmHg by PAI-1 12580 genotype (GG, GT and TT separately) in 587 critically ill Caucasians with SIRS.
- Figure 9B.: shows days alive and free of acute lung injury and PₐO₂/FᵢO₂ < 300 mmHg by PAI-1 12580 genotype (GG/GT vs TT) for 587 Caucasians with SIRS.
- Figure 10A.: shows days alive and free of acute lung injury (ALI) and PₐO₂/FᵢO₂ (P/F) ratio greater than 300 mmHg by PAI-1 4G/5G/ (837) genotype (4G/4G, 4G/5G and 5G/5G separately) in 314 critically ill Caucasians with SIRS.
- Figure 10B.: shows days alive and free of acute lung injury and PₐO₂/FᵢO₂ < 300 mmHg by PAI-1 4G/5G allele (any 4G vs 5G/5G) in 314 critically ill Caucasians with SIRS.
- Figure 11A.: shows days alive and free of steroid use by PAI-1 12580 genotype in 587 critically ill Caucasians with SIRS. Patients with 2 copies of the 12580G allele (N = 98) had the fewest days alive and free of steroid use (15 days).
- Figure 11B.: shows days alive and free of steroid use by PAI-1 12580 genotype (GG/GT vs TT) for 587 critically ill Caucasians with SIRS.
- Figure 12B.: shows days alive and free of steroid use by PAI-1 4G/5G (837) genotype in critically ill Caucasians with SIRS.

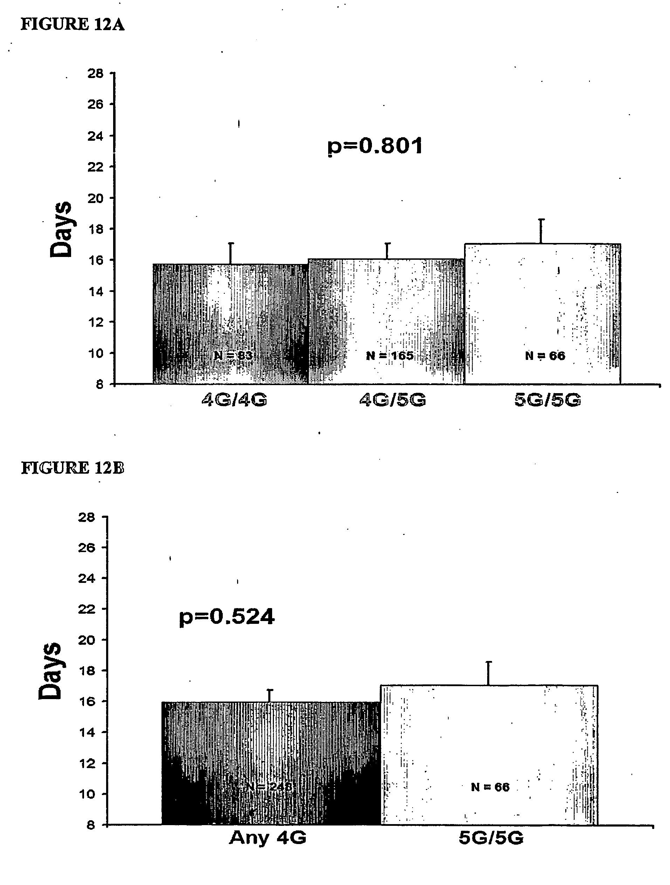

- Figure 12B.: shows days alive and free of steroid use by PAI-1 4G/5G (837) allele in 314 critically ill Caucasians with SIRS.

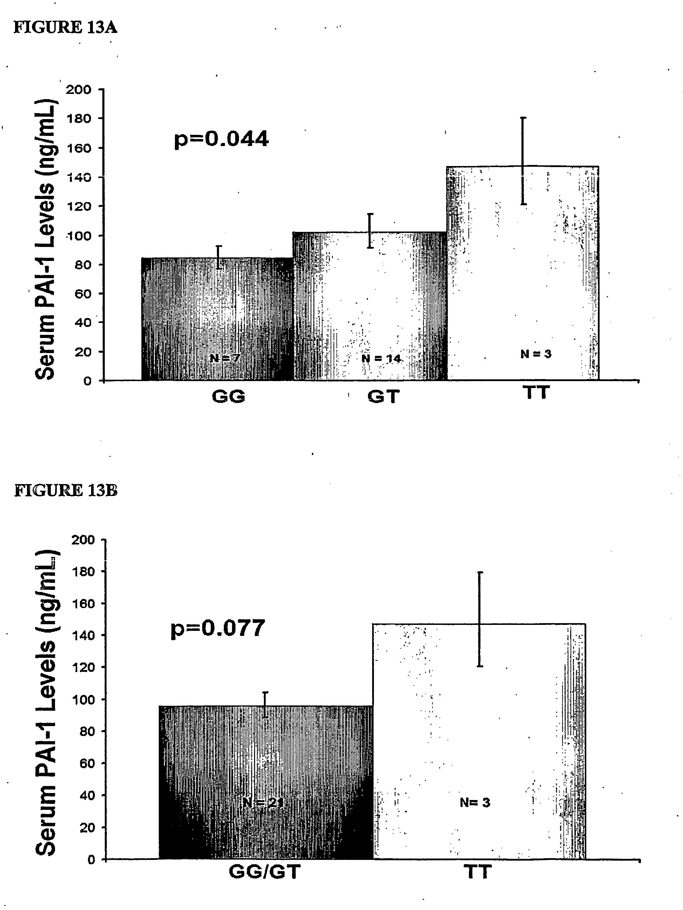

- Figure 13A.: shows mean pre-operative PAI-1 serum levels in 24 Caucasians who underwent on-pump cardiopulmonary bypass surgery grouped by PAI-1 12580 genotype (GG, GT and TT separately).
- Figure 13B.: shows mean pre-operative serum PAI-1 levels in 24 Caucasians who underwent on-pump cardiopulmonary bypass surgery grouped by PAI-1 12580 genotype (GG/GT vs TT).

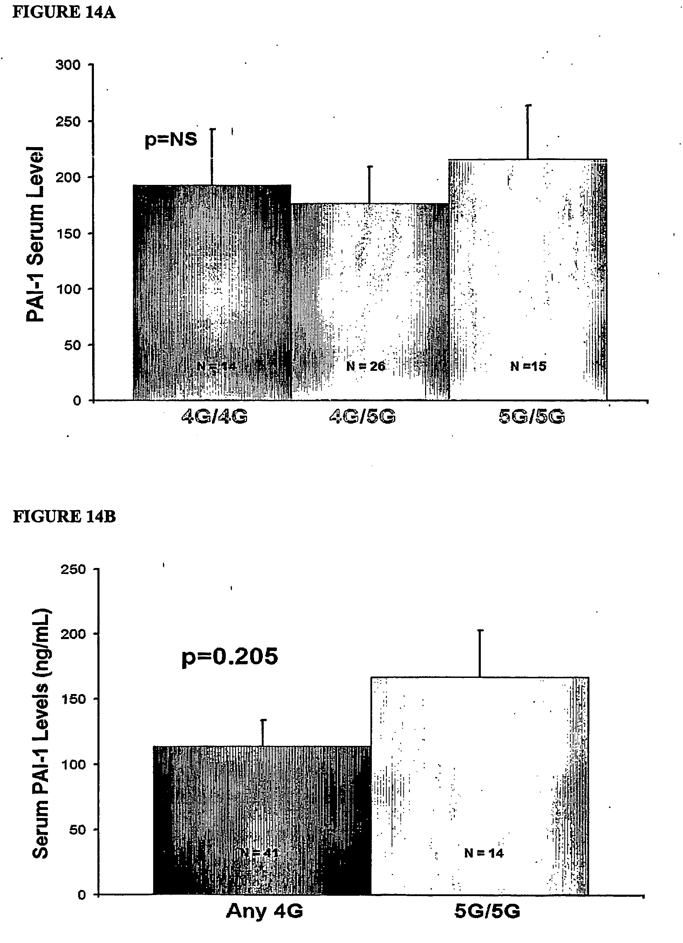

- Figure 14A.: shows serum PAI-1 levels measured 4 hours post-operatively in 55 Caucasians patients who underwent cardiopulmonary bypass surgery grouped by PAI-1 4G/5G.
- Figure 14B.: shows serum PAI-1 levels measured 4 hours post-operatively in 55 Caucasians who underwent on-pump cardiopulmonary bypass surgery grouped by PAI-1 4G/5G.

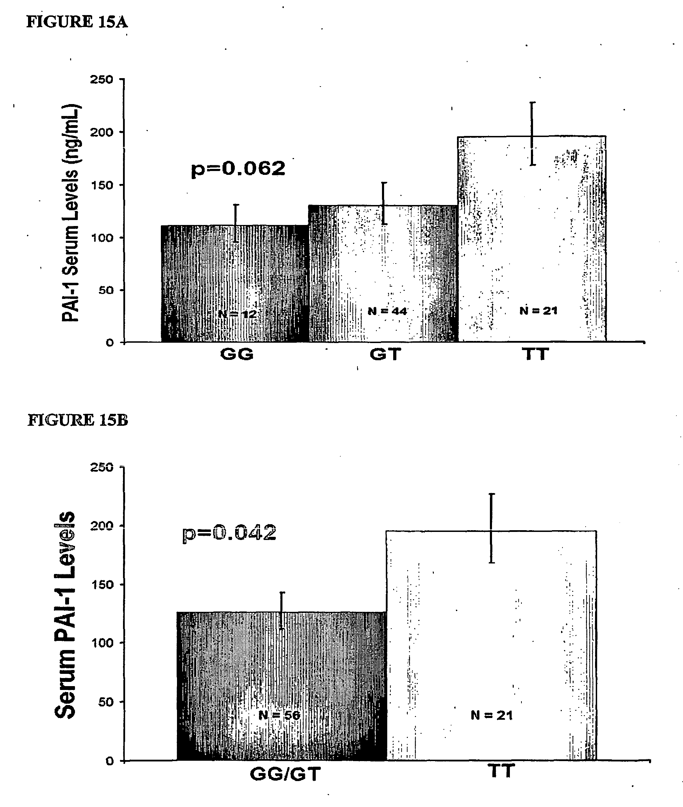

- Figure 15A.: shows mean serum PAI-1 levels of 67 Caucasians who underwent on-pump cardiopulmonary bypass surgery collected at 3 hours post surgery grouped by PAI-1 12580 genotype (TT, GT and GG separately).
- Figure 15B.: shows mean serum PAI-1 levels of 67 Caucasians who underwent on-pump cardiopulmonary bypass surgery collected 3 hours post-operatively grouped by 12580 GG/GT vs. TT.

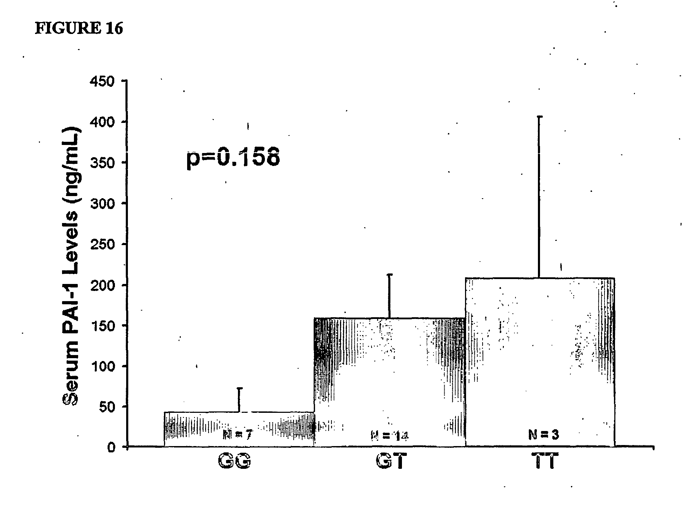

- Figure 16.: shows mean differences in serum PAI-1 levels from pre-operative to 3 hours post-operative in 24 Caucasians who underwent on-pump cardiopulmonary bypass surgery grouped by PAI-1 12580 GG, GT, and TT.

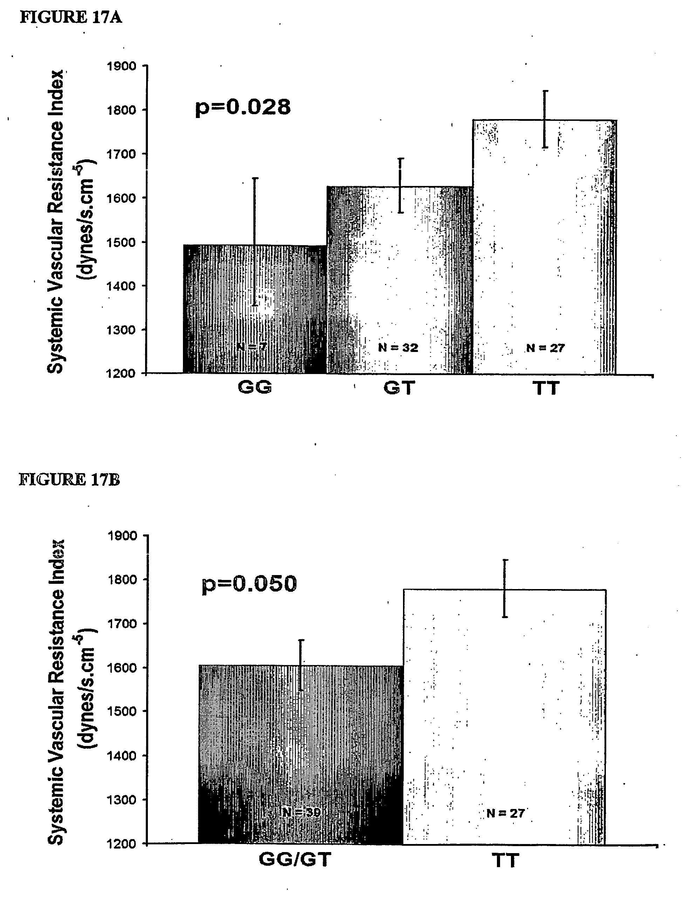

- Figure 17A.: shows systemic vascular resistance index (SVRI) 4 hours post-operatively in 66 Caucasians undergoing on-pump cardiopulmonary bypass surgery grouped by PAI-1 12580 genotype (GG, GT and TT separately).
- Figure 17B.: shows Systemic vascular resistance index (SVRI) 4 hours post-operatively in 66 Caucasians undergoing on-pump cardiopulmonary bypass surgery grouped by PAI-1 12580 genotype (GG/GT vs TT).

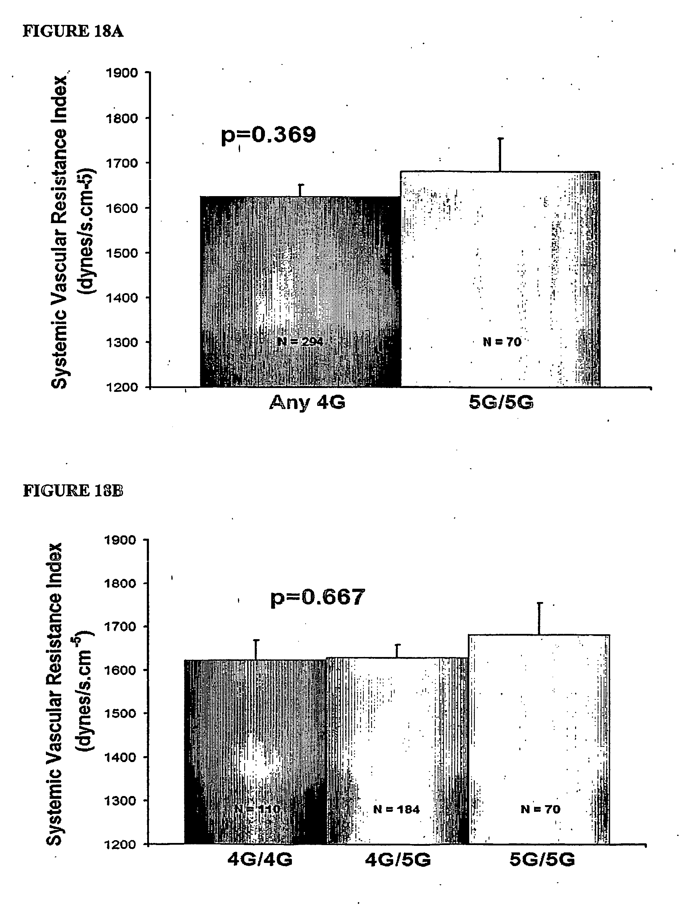

- Figure 18A.: shows the systemic vascular resistance index (SVRI) 4 hours post-operatively in 364 Caucasians undergoing on-pump cardiopulmonary bypass surgery grouped by PAI-1 4G/5G (837) genotype (4G/4G, 4G/5G and 5G/5G separately).
- Figure 18B.: shows the systemic vascular resistance index (SVRI) 4 hours post-operatively in 364 Caucasians undergoing on-pump cardiopulmonary bypass surgery grouped by PAI-1 4G/5G (837) allele (any 4G vs 5G/5G).

### DETAILED DESCRIPTION OF THE INVENTION

### 1. Definitions

In the description that follows, a number of terms are used extensively, the following definitions are provided to facilitate understanding of the invention.

"Genetic material" includes any nucleic acid and can be a deoxyribonucleotide or ribonucleotide polymer in either single or double-stranded form.

A "purine" is a heterocyclic organic compound containing fused pyrimidine and imidazole rings, and acts as the parent compound for purine bases, adenine (A) and guanine (G). "Nucleotides" are generally a purine (R) or pyrimidine (Y) base covalently linked to a pentose, usually ribose or deoxyribose, where the sugar carries one or more phosphate groups. Nucleic acids are generally a polymer of nucleotides joined by 3' 5' phosphodiester linkages. As used herein "purine" is used to refer to the purine bases, A and G, and more broadly to include the nucleotide monomers, deoxyadenosine-5' -phosphate and deoxyguanosine-5' -phosphate, as components of a polynucleotide chain.

A "pyrimidine" is a single-ringed, organic base that forms nucleotide bases, cytosine (C), thymine (T) and uracil (U). As used herein "pyrimidine" is used to refer to the pyrimidine bases, C, T and U, and more broadly to include the pyrimidine nucleotide monomers that along with purine nucleotides are the components of a polynucleotide chain.

A "polymorphic site" or "polymorphism site" or "polymorphism" or "single nucleotide polymorphism site" (SNP site) as used herein is the locus or position with in a given sequence at which divergence occurs. A "Polymorphism" is the occurrence of two or more forms of a gene or position within a gene (allele), in a population, in such frequencies that the presence of the rarest of the forms cannot be explained by mutation alone. The implication is that polymorphic alleles confer some selective advantage on the host. Preferred polymorphic sites have at least two alleles, each occurring at frequency of greater than 1%, and more preferably greater than 10% or 20% of a selected population. Polymorphism sites may be at known positions within a nucleic acid sequence or may be determined to exist using the methods described herein. Polymorphisms may occur in both the coding regions and the noncoding regions (for example, upstream promoters) of genes.

A "clade" is a group of haplotypes that are closely related phylogenetically. For example, if haplotypes are displayed on an phylogentic (evolutionary) tree a clade includes all haplotypes contained within the same branch.

As used herein "haplotype" is a set of alleles of closely linked loci on a chromosome that tend to be inherited together; commonly used in reference to the linked genes of the major histocompatibility complex.

As used herein "linkage disequilibrium" is the occurrence in a population of certain combinations of linked alleles in greater proportion than expected from the allele frequencies at the loci.

Numerous other sites have been identified as polymorphisms in the PAI-1 gene, where those polymorphisms are linked to the polymorphism at position 12580 of SEQ. ID NO: 1 and may therefore be indicative of patient prognosis. The following single polymorphism sites and combined polymorphism sites are linked to 12580 of SEQ. ID NO.: 1:
5645;
7121;
7437;
8070;
8406;
9463;
9466;
12219;
13889;
14440;
664 and 2037;
664 and 2362;
664 and 2852;
664 and 5834;
837 and 2037;
837 and 2362;
837 and 2852; and
837 and 5834.

It will be appreciated by a person of skill in the art that further linked single polymorphism sites and combined polymorphism sites could be determined. The haplotype of PAI-1 can be created by assessing the SNP's of PAI-1, in normal subjects using a program that has an expectation maximization algorithm (i.e. PHASE). A constructed haplotype of PAI-1 may be used to find combinations of SNP's that are in total linkage disequilibrium (LD) with 12580. Therefore, the haplotype of an individual could be determined by genotyping other SNP's that are in total LD with 12580. Linked single polymorphism sites or combined polymorphism sites may also be genotyped for assessing patient prognosis.

The following genotypes for single polymorphism sites and combined polymorphism sites may be indicative of a decreased likelihood of recovery from an inflammatory condition or indicative of severe cardiovascular or respiratory dysfunction in critically ill patients:
5645 T;
7121 G;
7437 T;
8070 A;
8406 C;
9463 G;
9466 T;
12219 C;
12580 G;
13889 C;
14440 A;
664 A and 2037 T;
664 A and 2362 -;
664 A and 2852 A;
664 A and 5834 A;
837 - and 2037 T;
837 - and 2362 -;
837 - and 2852 A; and
837 - and 5834 A.

Whereas the following genotypes for single polymorphism sites and combined polymorphism sites may be indicative of a increased likelihood of recovery from an inflammatory condition or indicative of less severe cardiovascular or respiratory dysfunction in critically ill patients:
5645 C;
7121 A;
7437 C;
8070 G;
8406 T;
9463 A;
9466 C;
12219 T;
12580 T;
13889 T;
14440 G;

| | | | | | |
|---|---|---|---|---|---|
| one of | 5878 G | and one of | 7365 T | and one of | 4588 T |
| | 7343 G | | 7729 + | | 5404 G |
| | 13605 A | | 7771 A | | 5686 A |
| | | | 12750 A | | 5984 A |
| | | | | | 11312 A; |

and

| | | | | | | | |
|---|---|---|---|---|---|---|---|
| one of | 2846 A | and | 10381 T | and one of | 7365 T | and one of | 4588 T |
| | 6821 T | and | 10381 T | | 7729 + | | 5404 G |
| | 9759 G | and | 10381 T | | 7771 A | | 5686 A |
| | | | | | 12750 A | | 5984 A |
| | | | | | | | 11312 A. |

(wherein a "+" represents an insertion and a "-" represents a deletion)

It will be appreciated by a person of skill in the art, that the numerical designations of the positions of polymorphisms within a sequence are relative to the specific sequence. Also the same positions may be assigned different numerical designations depending on the way in which the sequence is numbered and the sequence chosen, as illustrated by the alternative numbering of equivalent polymorphism in DAWSON *et al.* (1993) above. Furthermore, sequence variations within the population, such as insertions or deletions, may change the relative position and subsequently the numerical designations of particular nucleotides at and around a polymorphism site.

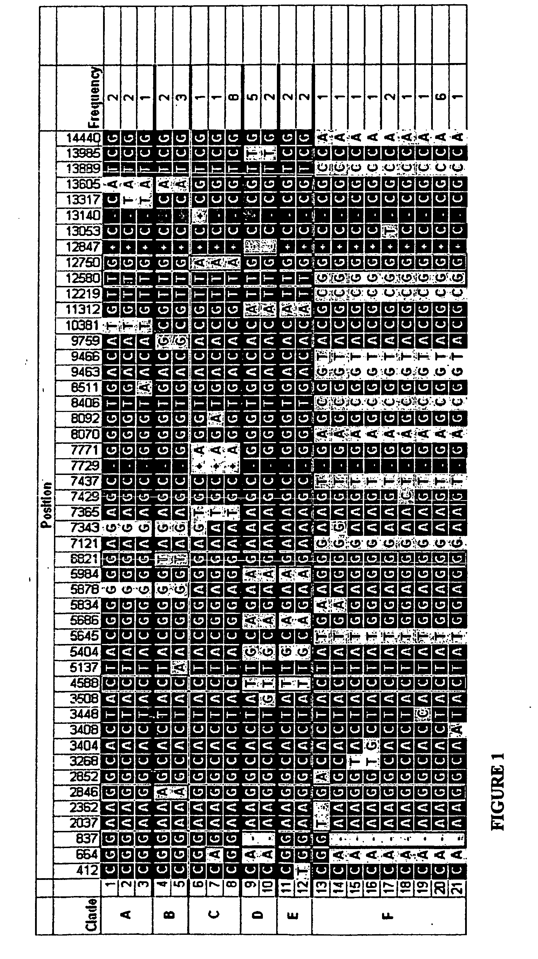

TABLE 1A below is representative of a *Homo sapiens* Plasminogen Activator Inhibitor- (PAI-1) or Serine-cysteine Proteinase Inhibitor clade E member 1 (SERPINE1)
gene sequence and comprises a sequence as listed in GenBank under accession number AF386492. The 4G/5G polymorphism of PAI-1 corresponds to the SNP described as -675 G/using the numbering system of DAWSON *et al.* (1993), which corresponds to position 837 in TABLE 1A (SEQ ID NO: 1). Polymorphism sites shown below in TABLE 1A are bolded and underlined. The polymorphism sites are represented by their major allele (dark cells in Figure 1). The major and minor alleles for each of the primary polymorphism sites of the PAI-1 gene are as follows:
at position 5445 the most common nucleotide (major allele) is **g** and the minor allele is **a**;
at position 837 the most common nucleotide (major allele) is **g** and the minor allele is -;
at position 5445 the most common nucleotide (major allele) is **c** and the minor allele is **t**;
at position 7121 the most common nucleotide (major allele) is **a** and the minor allele is **g**;
at position 7437 the most common nucleotide (major allele) is **c** and the minor allele is **t**;
at position 8070 the most common nucleotide (major allele) is **g** and the minor allele is **a**;
at position 8406 the most common nucleotide (major allele) is **t** and the minor allele is **c**;
at position 9463 the most common nucleotide (major allele) is **a** and the minor allele is **g**;
at position 9466 the most common nucleotide (major allele) is **c** and the minor allele is **t**;
at position 12219 the most common nucleotide (major allele) is **t** and the minor allele is **c**;
at position 12580 the most common nucleotide (major allele) is **t** and the minor allele is **g**;
at position 13889 the most common nucleotide (major allele) is **t** and the minor allele is **c**;
at position 14440 the most common nucleotide (major allele) is **g** and the minor allele is **a**.

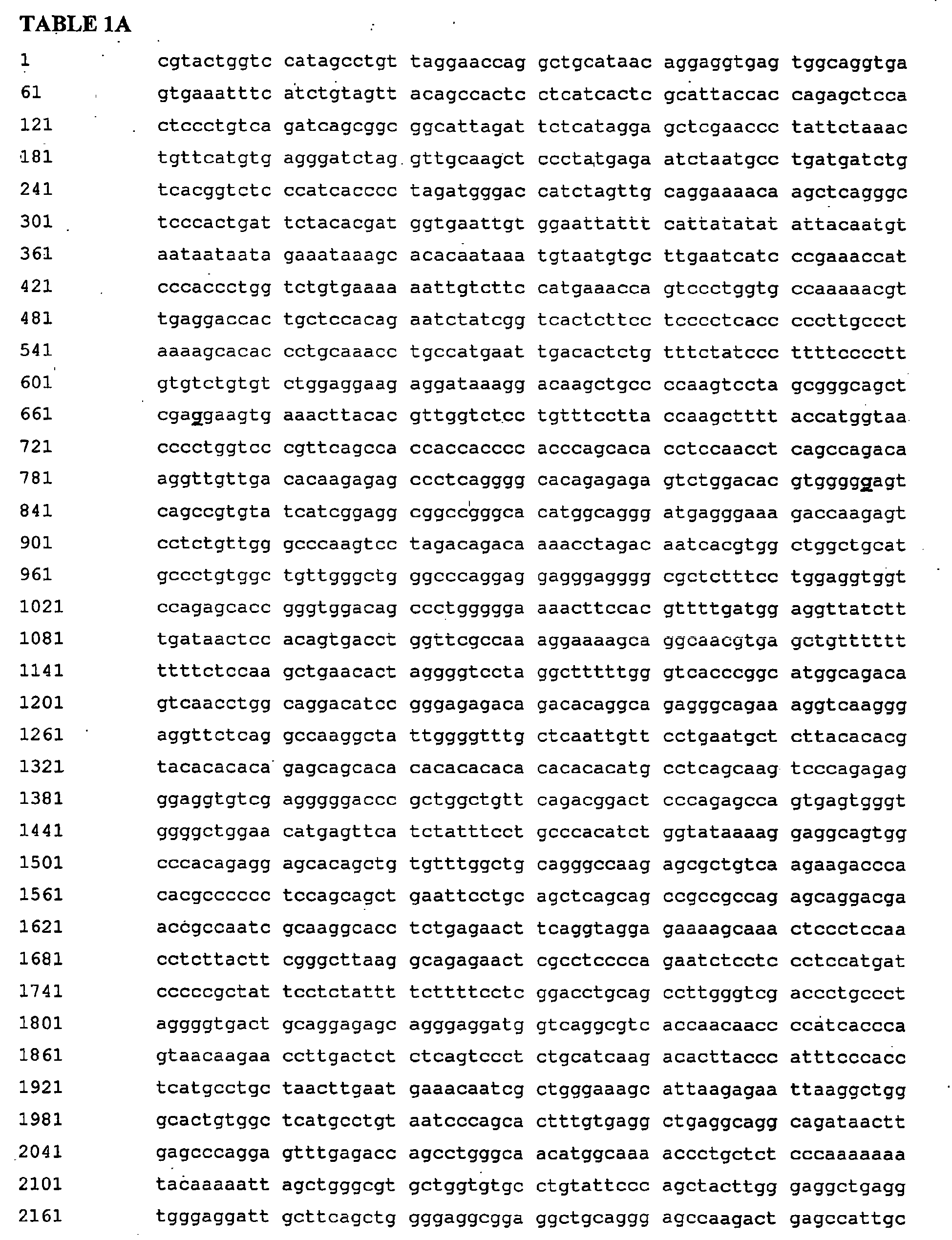

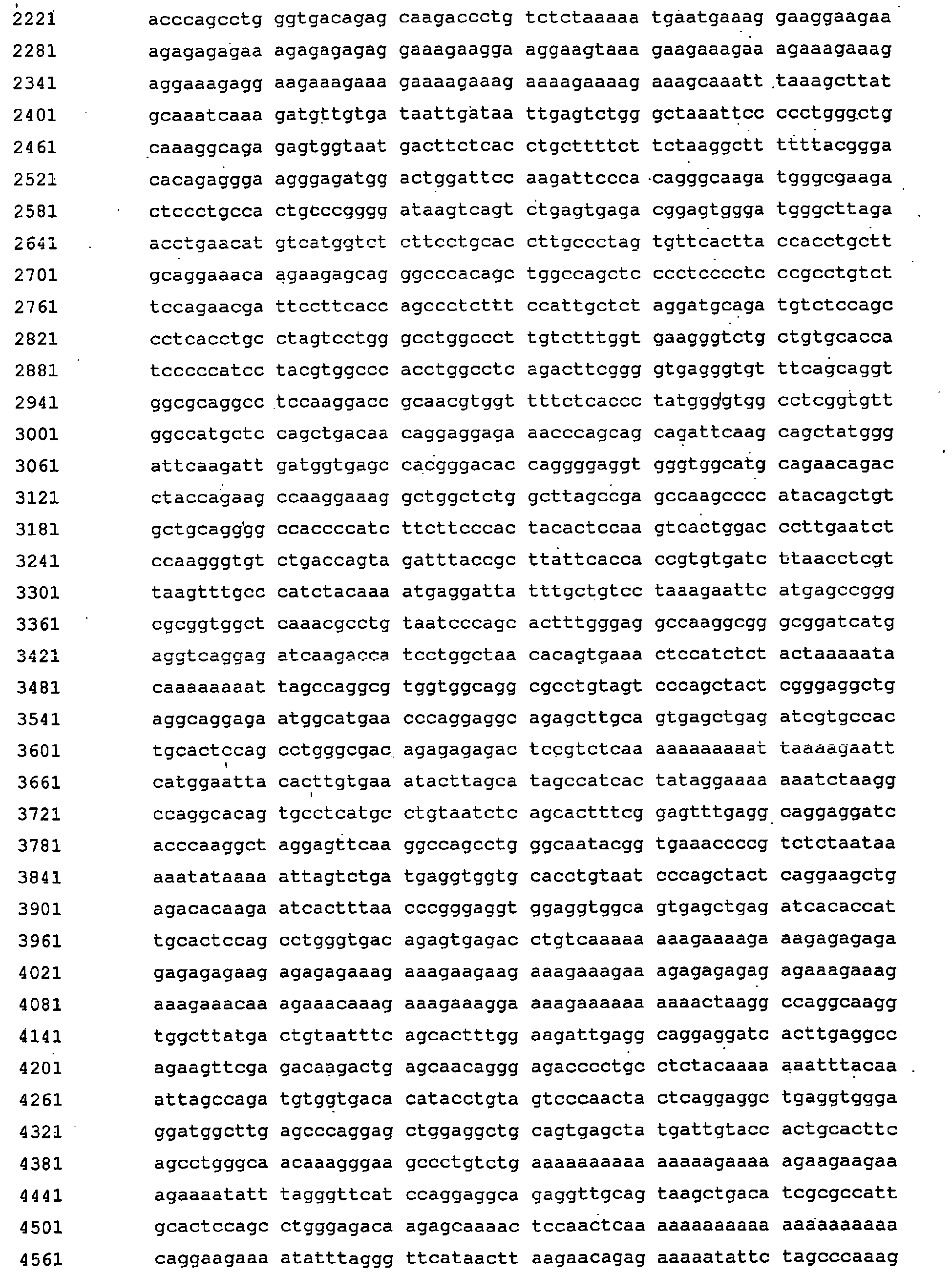

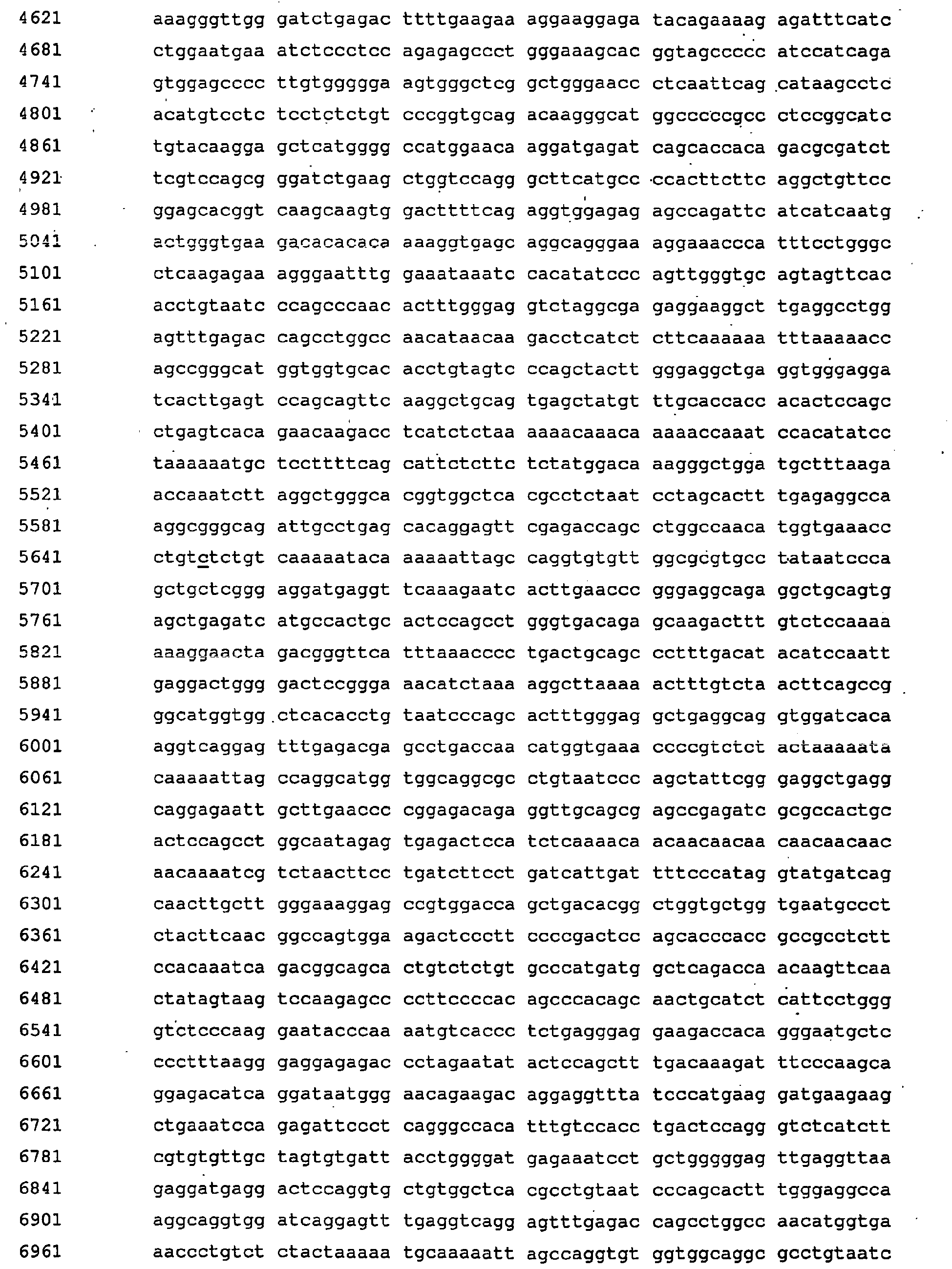

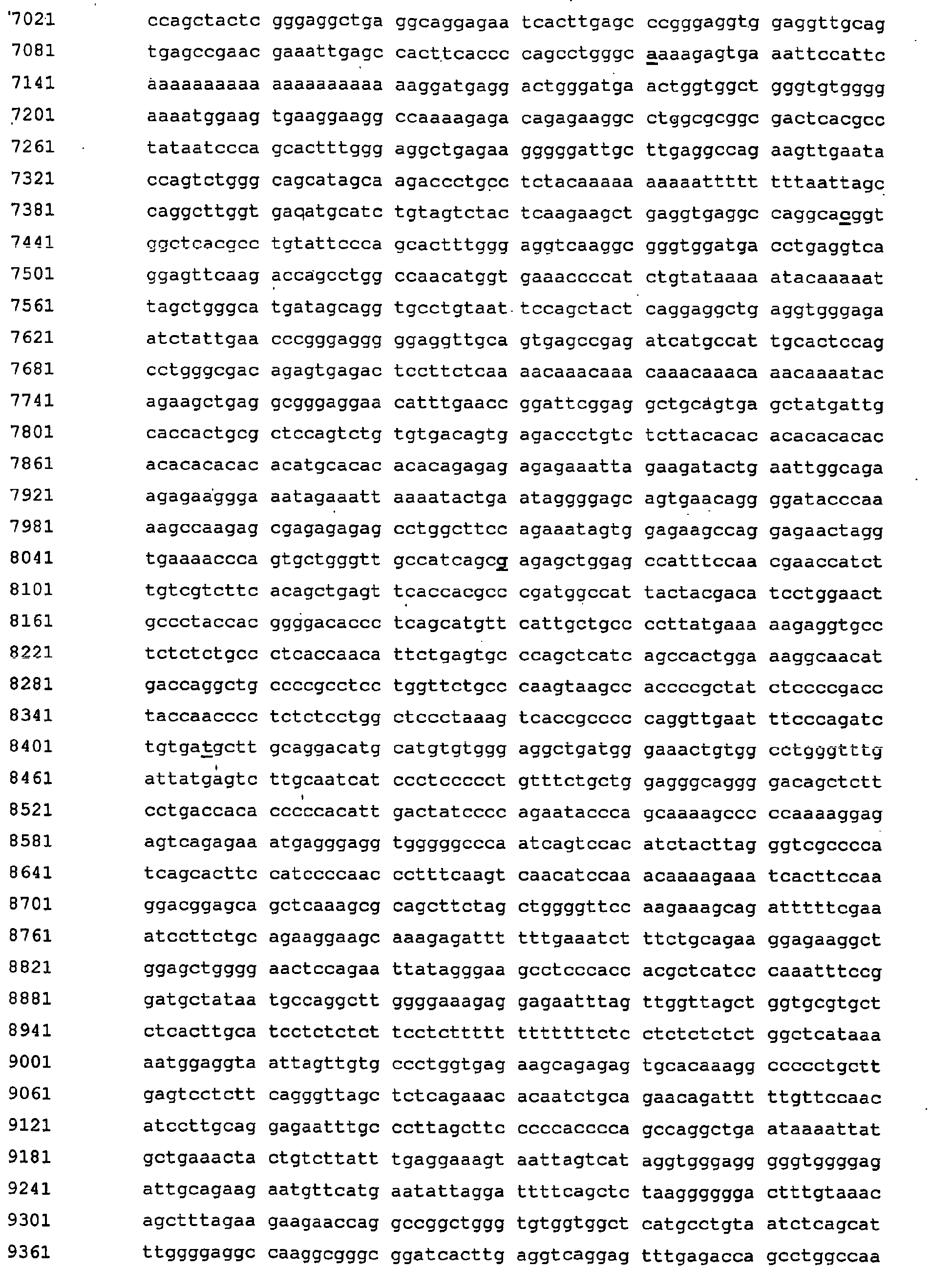

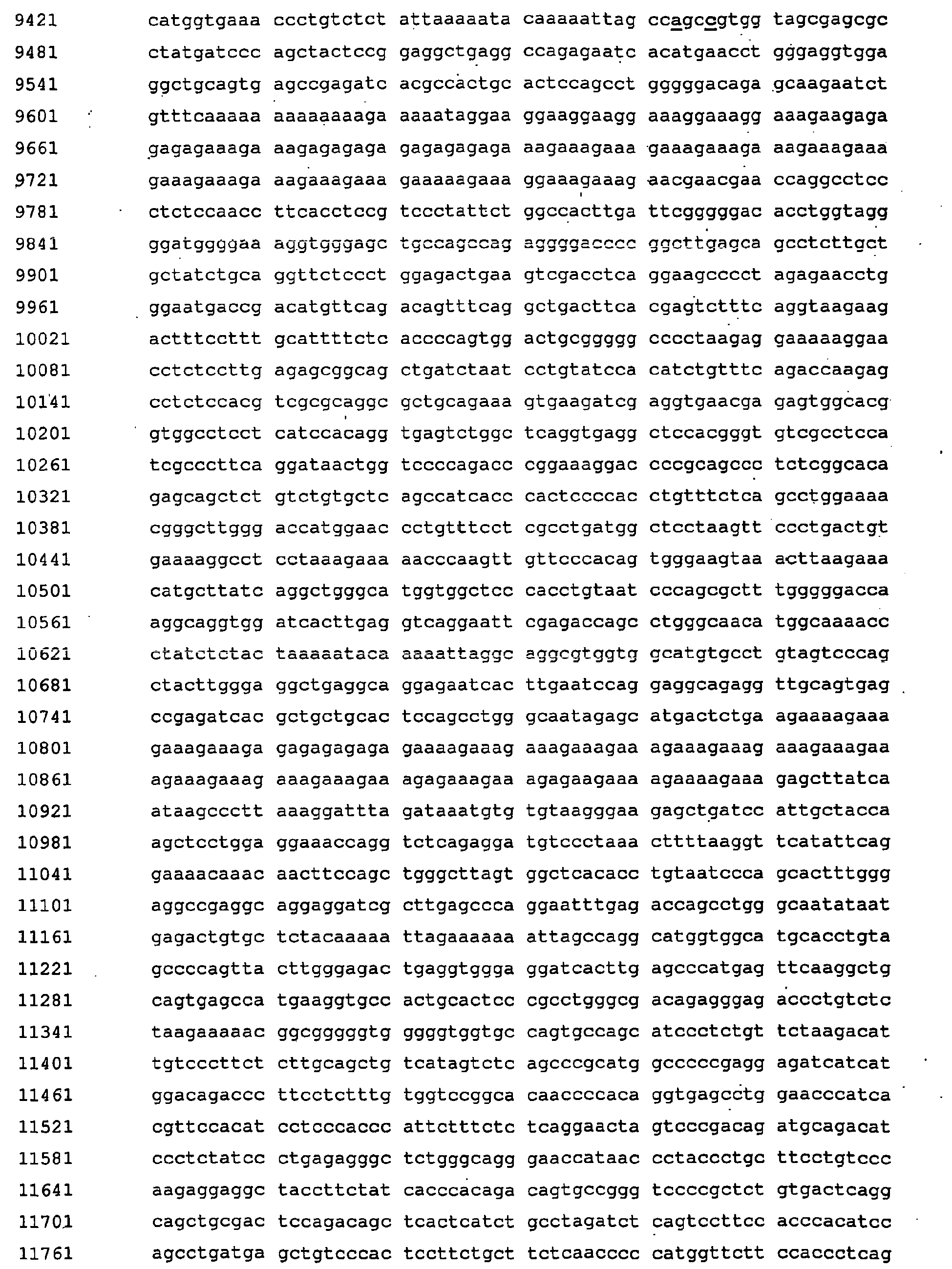

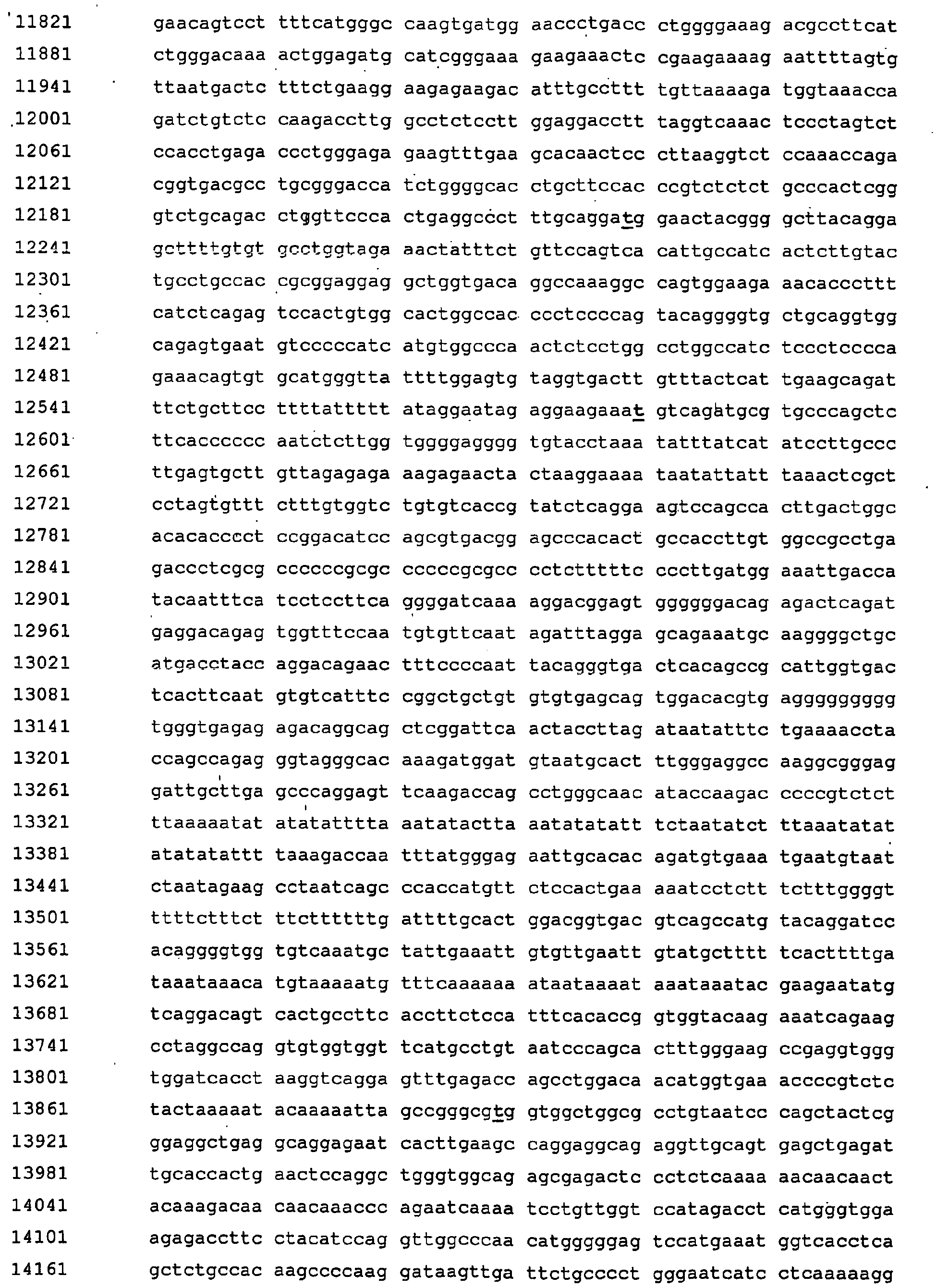

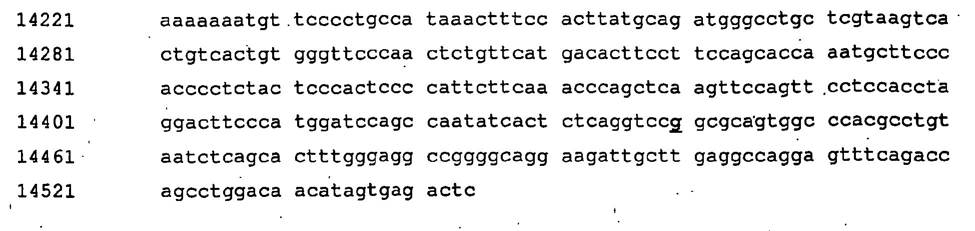

### TABLE 1B

The sequences shown in TABLE 1B, are sequence fragments taken from the PAI-1 sequence shown in TABLE 1A above. Furthermore, SEQ ID NO.: 2 corresponds to positions12571-12590 in TABLE 1A above. The nucleotide **t**, at position 10 in SEQ ID NO.: 2 corresponds to the nucleotide found at position 12980 of SEQ ID NO.: 1. In all of the sequences found in TABLE 2B below the polymorphism represented by the bold and underlined **t** may be substituted by a **g**. In SEQ ID NOs.: 3-11 in TABLE 2B, the bold and underlined **t** corresponds to the nucleotide found at position 12580 of SEQ ID NO.: 1. Due to the potential variability in PAI-1 sequence, the sequence motifs below may be useful in identifying PAI-1 sequences from a patient for use in genotype determination. For Example, patient sequences that form alignments with the below motifs (SEQ ID NO.: 3-11) may indicate that the patient sequence is a PAI-1 sequence and that nucleotide found at the position occupied by the bold and underlined **t** corresponds to the polymorphism at position 12580 of SEQ ID NO.: 1 and is therefore suitable for genotype determination. A similar strategy may be applied to the other polymorphism sites identified herein.

| **SEQ ID. NO.** | **SEQUENCE** |
|---|---|
| SEQ ID. NO. 2 | aggaagaaa**t** gtcagatgcg |
| SEQ ID. NO. 3 | aggaatagag gaagaaa**t**gt |
| SEQ ID. NO. 4 | **t**gtcagatgc gtgcccagct |
| SEQ ID. NO. 5 | aggaagaaa**t** gtcagatgcg tgcccagctc |
| SEQ ID. NO. 6 | ataggaatag aggaagaaa**t** |
| SEQ ID. NO. 7 | ttttattttt ataggaatag aggaagaaa**t** |
| SEQ ID. NO. 8 | ttctgcttcc ttttattttt ataggaatag aggaagaaa**t** |
| SEQ ID. NO. 9 | |
| SEQ ID. NO. 10 | gaagaaa**t**gt cagatg |
| SEQ ID. NO. 11 | aagaaa**t**gtc ag |

An "allele" is defined as any one or more alternative forms of a given gene. In a diploid cell or organism the members of an allelic pair (i.e. the two alleles of a given gene) occupy corresponding positions (loci) on a pair of homologous chromosomes and if these alleles are genetically identical the cell or organism is said to be "homozygous", but if genetically different the cell or organism is said to be "heterozygous" with respect to the particular gene.

A "gene" is an ordered sequence of nucleotides located in a particular position on a particular chromosome that encodes a specific functional product and may include untranslated and untranscribed sequences in proximity to the coding regions. Such non-coding sequences may contain regulatory sequences needed for transcription and translation of the sequence or introns etc.

A "genotype" is defined as the genetic constitution of an organism, usually in respect to one gene or a few genes or a region of a gene relevant to a particular context (i.e. the genetic loci responsible for a particular phenotype).

A "phenotype" is defined as the observable characters of an organism.

A "single nucleotide polymorphism" (SNP) occurs at a polymorphic site occupied by a single nucleotide, which is the site of variation between allelic sequences. The site is usually preceded by and followed by highly conserved sequences of the allele (e.g., sequences that vary in less than 1/100 or 1/1000 members of the populations). A single nucleotide polymorphism usually arises due to substitution of one nucleotide for another at the polymorphic site. A "transition" is the replacement of one purine by another purine or one pyrimidine by another pyrimidine. A "transversion" is the replacement of a purine by a pyrimidine or vice versa. Single nucleotide polymorphisms can also arise from a deletion (represented by "-" or "*del*") of a nucleotide or an insertion (represented by "+" or "*ins*") of a nucleotide relative to a reference allele. Furthermore, it would be appreciated by a person of skill in the art, that an insertion or deletion within a given sequence could alter the relative position and therefore the position number of another polymorphism within the sequence.

A "systemic inflammatory response syndrome" or (SIRS) is defined as including both septic (i.e. sepsis or septic shock) and non-septic systemic inflammatory response (i.e. post operative). "SIRS" is further defined according to ACCP (American College of Chest Physicians) guidelines as the presence of two or more of A) temperature > 38°C or < 36°C, B) heart rate > 90 beats per minute, C) respiratory rate > 20 breaths per minute, and D) white blood cell count > 12,000 per mm3 or < 4,000 mm3. In the following description, the presence of two, three, or four of the "SIRS" criteria were scored each day over the 28 day observation period.

"Sepsis" is defined as the presence of at least two "SIRS" criteria and known or suspected source of infection. Septic shock was defined as sepsis plus one new organ failure by Brussels criteria plus need for vasopressor medication.

Patient outcome or prognosis as used herein refers the ability of a patient to recover from an inflammatory condition. An inflammatory condition, may be selected from the group consisting of: sepsis, septicemia, pneumonia, septic shock, systemic inflammatory response syndrome (SIRS), Acute Respiratory Distress Syndrome (ARDS), acute lung injury, infection, pancreatitis, bacteremia, peritonitis, abdominal abscess, inflammation due to trauma, inflammation due to surgery, chronic inflammatory disease, ischemia, ischemia-reperfusion injury of an organ or tissue, tissue damage due to disease, tissue damage due to chemotherapy or radiotherapy, and reactions to ingested, inhaled, infused, injected, or delivered substances, glomerulonephritis, bowel infection, opportunistic infections, and for patients undergoing major surgery or dialysis, patients who are immunocompromised, patients on immunosuppressive agents, patients with HIV/AIDS, patients with suspected endocarditis, patients with fever, patients with fever of unknown origin, patients with cystic fibrosis, patients with diabetes mellitus, patients with chronic renal failure, patients with bronchiectasis, patients with chronic obstructive lung disease, chronic bronchitis, emphysema, or asthma, patients with febrile neutropenia, patients with meningitis, patients with septic arthritis, patients with urinary tract infection, patients with necrotizing fasciitis, patients with other suspected Group A streptococcus infection, patients who have had a splenectomy, patients with recurrent or suspected enterococcus infection, other medical and surgical conditions associated with increased risk of infection, Gram positive sepsis, Gram negative sepsis, culture negative sepsis, fungal sepsis, meningococcemia, post-pump syndrome, cardiac stun syndrome, myocardial infarction, stroke, congestive heart failure, hepatitis, epiglotittis, E. coli 0157:H7, malaria, gas gangrene, toxic shock syndrome, mycobacterial tuberculosis, Pneumocystic carinii, pneumonia, Leishmaniasis, hemolytic uremic syndrome/thrombotic thrombocytopenic purpura, Dengue hemorrhaigic fever, pelvic inflammatory disease, Legionella, Lyme disease, Influenza A, Epstein-Barr virus, encephalitis, inflammatory diseases and autoimmunity including Rheumatoid arthritis, osteoarthritis, systemic lupus erythematosus, inflammatory bowel disease, idiopathic pulmonary fibrosis, sarcoidosis, hypersensitivity pneumonitis, systemic vasculitis, Wegener's granulomatosis, transplants including heart, liver, lung kidney bone marrow, graft-versus-host disease, transplant rejection, sickle cell anemia, nephrotic syndrome, toxicity of agents such as OKT3, cytokine therapy, and cirrhosis.

Assessing patient outcome or prognosis may be accomplished by various methods. For Example, an "APACHE II" score is defined as Acute Physiology And Chronic Health Evaluation and herein was calculated on a daily basis from raw clinical and laboratory variables. (VINCENT et al. Scoring systems for assessing organ dysfunction and survival. Critical Care Clinics. 16:353-366, 2000) summarize APACHE score as follows "First developed in 1981 by KNAUS WA et al. (Chest (1991) 100(6):1619-36), the APACHE score has become the most commonly used survival prediction model in ICUs worldwide. The APACHE II score, a revised and simplified version of the original prototype, uses a point score based on initial values of 12 routine physiologic measures, age, and previous health status to provide a general measure of severity of disease. The values recorded are the worst values taken during the patient's first 24 hours in the ICU. The score is applied to one of 34 admission diagnoses to estimate a disease-specific probability of mortality (APACHE II predicted risk of death). The maximum possible APACHE II score is 71, and high scores have been well correlated with mortality. The APACHE II score has been widely used to stratify and compare various groups of critically ill patients, including patients with sepsis, by severity of illness on entry into clinical trials."

A "Brussels score" score is a method for evaluating organ dysfunction as compared to a baseline. If the Brussels score is 0 (ie. moderate, severe, or extreme), then organ failure was recorded as present on that particular day (see TABLE 1C below). In the following description, to correct for deaths during the observation period, days alive and free of organ failure (DAF) were calculated as previously described. For example, acute lung injury was calculated as follows. Acute lung injury is defined as present when a patient meets all of these four criteria. 1) Need for mechanical ventilation, 2) Bilateral pulmonary infiltrates on chest X-ray consistent with acute lung injury, 3) PaO₂/FiO₂ ratio is less than 300, 4) No clinical evidence of congestive heart failure or if a pulmonary artery catheter is in place for clinical purposes, a pulmonary capillary wedge pressure less than 18 mm Hg (1). The severity of acute lung injury is assessed by measuring days alive and free of acute lung injury over a 28 day observation period. Acute lung injury is recorded as present on each day that the person has moderate, severe or extreme dysfunction as defined in the Brussels score. Days alive and free of acute lung injury is calculated as the number of days after onset of acute lung injury that a patient is alive and free of acute lung injury over a defined observation period (28 days). Thus, a lower score for days alive and free of acute lung injury indicates more severe acute lung injury. The reason that days alive and free of acute lung injury is preferable to simply presence for absence of acute lung injury, is that acute lung injury has a high acute mortality and early death (within 28 days) precludes calculation of the presence or absence of acute lung injury in dead patients. The cardiovascular, renal, neurologic, hepatic and coagulation dysfunction were similarly defined as present on each day that the person had moderate, severe or extreme dysfunction as defined by the Brussels score. Days alive and free of steroids are days that a person is alive and is not being treated with exogenous corticosteroids (e.g. hydrocortisone, prednisone, methylprednisolone). Days alive and free of pressors are days that a person is alive and not being treated with intravenous vasopressors (e.g. dopamine, norepinephrine, epinephrine, phenylephrine). Days alive and free of an International Normalized Ratio (INR) > 1.5 are days that a person is alive and does not have an INR > 1.5.

**TABLE 1C**

| **Brussels Organ Dysfunction Scoring System** | | | | | |
|---|---|---|---|---|---|
| ORGANS | Free of Organ Dysfunction | | Clinically Significant Organ Dysfunction | | |
| | Normal | Mild | Moderate | Severe | Extreme |
| **DAF ORGAN DYSFUNCTION SCORE** | **1** | | **0** | | |
| Cardiovascular Systolic BP (mmHg) | >90 | ≤90 Responsive to fluid | ≤90 Unresponsive to fluid | ≤90 plus p ≤7.3 | ≤90 plus pH ≤7.2 |
| Pulmonary PₐO₂/F₁O₂ (mmHg) | >400 | 400-301 | 300-201 Acute lung injury | 200-101 ARDS | ≤100 Severe ARDS |
| Renal Creatinine (mg/dL) | <1.5 | 1.5-1.9 | 2.0-3.4 | 3.5-4.9 | ≥5.0 |
| Hepatic Bilirubin (mg/dL) | <1.2 | 1.2-1.9 | 2.0-5.9 | 6.0-11.9 | ≥12 |
| Hematologic Platelets (x10⁵/mm³) | >120 | 120-81 | 80-51 | 50-21 | ≤20 |
| Neurologic (Glascow Score) | 15 | 14-13 | 12-10 | 9-6 | ≤5 |
| Round Table Conference on Clinical Trials for the Treatment of Sepsis Brussels, March 12-14, 1994. | | | | | |

Analysis of variance (ANOVA) is a standard statistical approach to test for statistically significant differences between sets of measurements.

The Fisher exact test is a standard statistical approach to test for statistically significant differences between rates and proportions of characteristics measured in different groups.

### 2. General Methods

One aspect of the invention may involve the identification of patients or the selection of patients that are either at risk of developing and inflammatory condition or the identification of patients who already have an inflammatory condition. For example, patients who have undergone major surgery or scheduled for or contemplating major surgery may be considered as being at risk of developing an inflammatory condition. Furthermore, patients may be determined as having an inflammatory condition using diagnostic methods and clinical evaluations known in the medical arts. An inflammatory condition, may be selected from the group consisting of: sepsis, septicemia, pneumonia, septic shock, systemic inflammatory response syndrome (SIRS), Acute Respiratory Distress Syndrome (ARDS), acute lung injury, infection, pancreatitis, bacteremia, peritonitis, abdominal abscess, inflammation due to trauma, inflammation due to surgery, chronic inflammatory disease, ischemia, ischemia-reperfusion injury of an organ or tissue, tissue damage due to disease, tissue damage due to chemotherapy or radiotherapy, and reactions to ingested, inhaled, infused, injected, or delivered substances, glomerulonephritis, bowel infection, opportunistic infections, and for patients undergoing major surgery or dialysis, patients who are immunocompromised, patients on immunosuppressive agents, patients with HIV/AIDS, patients with suspected endocarditis, patients with fever, patients with fever of unknown origin, patients with cystic fibrosis, patients with diabetes mellitus, patients with chronic renal failure, patients with bronchiectasis, patients with chronic obstructive lung disease, chronic bronchitis, emphysema, or asthma, patients with febrile neutropenia, patients with meningitis, patients with septic arthritis, patients with urinary tract infection, patients with necrotizing fasciitis, patients with other suspected Group A streptococcus infection, patients who have had a splenectomy, patients with recurrent or suspected enterococcus infection, other medical and surgical conditions associated with increased risk of infection, Gram positive sepsis, Gram negative sepsis, culture negative sepsis, fungal sepsis, meningococcemia, post-pump syndrome, cardiac stun syndrome, myocardial infarction, stroke, congestive heart failure, hepatitis, epiglotittis, E. coli 0157:H7, malaria, gas gangrene, toxic shock syndrome, mycobacterial tuberculosis, Pneumocystic carinii, pneumonia, Leishmaniasis, hemolytic uremic syndrome/thrombotic thrombocytopenic purpura, Dengue hemorrhaigic fever, pelvic inflammatory disease, Legionella, Lyme disease, Influenza A, Epstein-Barr virus, encephalitis, inflammatory diseases and autoimmunity including Rheumatoid arthritis, osteoarthritis, systemic lupus erythematosus, inflammatory bowel disease, idiopathic pulmonary fibrosis, sarcoidosis, hypersensitivity pneumonitis, systemic vasculitis, Wegener's granulomatosis, transplants including heart, liver, lung kidney bone marrow, graft-versus-host disease, transplant rejection, sickle cell anemia, nephrotic syndrome, toxicity of agents such as OKT3, cytokine therapy, and cirrhosis.

Once a patient is identified as being at risk for developing or having an inflammatory condition, then genetic sequence information may be obtained from the patient. Or alternatively genetic sequence information may already have been obtained from the patient. For example; a patient may have already provided a biological sample for other purposes or may have even had their genetic sequence determined in whole or in part and stored for future use. Genetic sequence information may be obtained in numerous different ways and may involve the collection of a biological sample that contains genetic material. Particularly, genetic material, containing the sequence or sequences of interest. Many methods are known in the art for collecting bodily samples and extracting genetic material from those samples. Genetic material can be extracted from blood, tissue and hair and other samples. There are many known methods for the separate isolation of DNA and RNA from biological material. Typically, DNA may be isolated from a biological sample when first the sample is lysed and then the DNA is isolated from the lysate according to any one of a variety of multi-step protocols, which can take varying lengths of time. DNA isolation methods may involve the use of phenol (SAMBROOK, J. et al., "Molecular Cloning", Vol. 2, pp. 9.14-9.23, Cold Spring Harbor Laboratory Press (1989) and AUSUBEL, FM. et al., "Current Protocols in Molecular Biology", Vol. 1, pp. 2.2.1-2.4.5, John Wiley & Sons, Inc. (1994)). Typically, a biological sample is lysed in a detergent solution and the protein component of the lysate is digested with proteinase for 12-18 hours. Next, the lysate is extracted with phenol to remove most of the cellular components, and the remaining aqueous phase is processed further to isolate DNA. In another method, described in VAN NESS et al. (U.S. Pat # 5,130,423), non-corrosive phenol derivatives are used for the isolation of nucleic acids. The resulting preparation is a mix of RNA and DNA.

Other methods for DNA isolation utilize non-corrosive chaotropic agents. These methods, which are based on the use of guanidine salts, urea and sodium iodide, involve lysis of a biological sample in a chaotropic aqueous solution and subsequent precipitation of the crude DNA fraction with a lower alcohol. The final purification of the precipitated, crude DNA fraction can be achieved by any one of several methods, including column chromatography (ANALECTS, (1994) Vol 22, No. 4, Pharmacia Biotech), or exposure of the crude DNA to a polyanion-containing protein as described in KOLLER (U.S. Pat. # 5,128,247).

Yet another method of DNA isolation, which is described by BOTWELL DDL (Anal. Biochem. (1987) 162:463-465) involves lysing cells in 6M guanidine hydrochloride, precipitating DNA from the lysate at acid pH by adding 2.5 volumes of ethanol, and washing the DNA with ethanol.

Numerous other methods are known in the art to isolate both RNA and DNA, such as the one described by CHOMCZYNSKI (U.S. Pat. # 5,945,515), whereby genetic material can be extracted efficiently in as little as twenty minutes. EVANS and HUGH (U.S. Pat. # 5,989,431) describe methods for isolating DNA using a hollow membrane filter.

Once a patient's genetic sequence information has been obtained from the patient it may then be further analyzed to detect or determine the identity or genotype of one or more polymorphisms in the PAI-1 gene. Provided that the genetic material obtained, contains the sequence of interest. Particularly, a person may be interested in determining the PAI-1 promoter genotype of a patient of interest, where the genotype includes a nucleotide corresponding to position 12580 or SEQ ID NO.: 1 or position 8 of SEQ ID NO.: 2. The sequence of interest may also include other PAI-1 gene polymorphisms or may also contain some of the sequence surrounding the polymorphism of interest. Detection or determination of a nucleotide identity or the genotype of the single nucleotide polymorphism(s) or other polymorphism, may be accomplished by any one of a number methods or assays known in the art, including but not limited to the following:
Restriction Fragment Length Polymorphism (RFLP) strategy - An RFLP gel-based analysis can be used to distinguish between alleles at polymorphic sites within a gene. Briefly, a short segment of DNA (typically several hundred base pairs) is amplified by PCR. Where possible, a specific restriction endonuclease is chosen that cuts the short DNA segment when one variant allele is present but does not cut the short DNA segment when the other allele variant is present. After incubation of the PCR amplified DNA with this restriction endonuclease, the reaction products are then separated using gel electrophoresis. Thus, when the gel is examined the appearance of two lower molecular weight bands (lower molecular weight molecules travel farther down the gel during electrophoresis) indicates that the initial DNA sample had the allele which could be cut by the chosen restriction endonuclease. In contrast, if only one higher molecular weight band is observed (at the molecular weight of the PCR product) then the initial DNA sample had the allele variant that could not be cut by the chosen restriction endonuclease. Finally, if both the higher molecular weight band and the two lower molecular weight bands are visible then the initial DNA sample contained both alleles, and therefore the patient was heterozygous for this single nucleotide polymorphism;
Sequencing - For example the Maxam-Gilbert technique for sequencing (MAXAM AM. and GILBERT W. Proc. Natl. Acad. Sci. USA (1977) 74(4):560-564) involves the specific chemical cleavage of terminally labelled DNA. In this technique four samples of the same labeled DNA are each subjected to a different chemical reaction to effect preferential cleavage of the DNA molecule at one or two nucleotides of a specific base identity. The conditions are adjusted to obtain only partial cleavage, DNA fragments are thus generated in each sample whose lengths are dependent upon the position within the DNA base sequence of the nucleotide(s) which are subject to such cleavage. After partial cleavage is performed, each sample contains DNA fragments of different lengths, each of which ends with the same one or two of the four nucleotides. In particular, in one sample each fragment ends with a C, in another sample each fragment ends with a C or a T, in a third sample each ends with a G, and in a fourth sample each ends with an A or a G. When the products of these four reactions are resolved by size, by electrophoresis on a polyacrylamide gel, the DNA sequence can be read from the pattern of radioactive bands. This technique permits the sequencing of at least 100 bases from the point of labeling. Another method is the dideoxy method of sequencing was published by Sanger *et al.* (SANGER et al. Proc. Natl. Acad. Sci. USA (1977) 74(12):5463-5467). The Sanger method relies on enzymatic activity of a DNA polymerase to synthesize sequence-dependent fragments of various lengths. The lengths of the fragments are determined by the random incorporation of dideoxynucleotide base-specific terminators. These fragments can then be separated in a gel as in the Maxam-Gilbert procedure, visualized, and the sequence determined. Numerous improvements have been made to refine the above methods, and to automate the sequencing procedures. Similary, RNA sequencing methods are also known. For example, reverse transcriptase with dideoxy-nucleotides have been used to sequence encephalomyocarditis virus RNA (ZIMMERN D. and KAESBERG P. Proc. Natl. Acad. Sci. USA (1978) 75(9):4257-4261). MILLS DR. and FRAMER FR. (Proc. Natl. Acad. Sci. USA (1979) 76(5):2232-2235) describe the use of Q.beta. replicase and the nucleotide analog inosine for sequencing RNA in a chain-termination mechanism. Direct chemical methods for sequencing RNA are also known (PEATTIE DA. Proc. Natl. Acad. Sci. USA (1979) 76(4):1760-1764). Other methods include those of DONIS-KELLER et al. (1977, Nucl. Acids Res. 4:2527-2538), SIMONCSITS A. et al. (Nature (1977) 269(5631):833-836), AXELROD VD. et al. (Nucl. Acids Res.(1978) 5(10):3549-3563), and FRAMER FR. and MILLS DR. (Proc. Natl. Acad. Sci. USA (1978) 75(11):5334-5338. Nucleic acid sequences can also be read by stimulating the natural fluoresce of a cleaved nucleotide with a laser while the single nucleotide is contained in a fluorescence enhancing matrix (U.S. Pat. # 5,674,743);
Hybridization methods for the identification of SNPs using hydridization techniques are described in the U.S. Pat. # 6,270,961 & 6,025,136;
A template-directed dye-terminator incorporation with fluorscent polarization-detection (TDI-FP) method is described by FREEMAN BD. et al. (J Mol Diagnostics (2002) 4(4):209-215) is described for large scale screening;
Oligonucleotide ligation assay (OLA) - is based on ligation of probe and detector oligonucleotides annealed to a polymerase chain reaction amplicon strand with detection by an enzyme immunoassay (VILLAHERMOSA ML. J Hum Virol (2001) 4(5):238-48; ROMPPANEN EL. Scand J Clin Lab Invest (2001) 61(2):123-9; IANNONE MA. et al. Cytometry (2000) 39(2):131-40);
Ligation-Rolling Circle Amplification (L-RCA) has also been successfully used for genotyping single nucleotide polymorphisms as described in QI X. et al. Nucleic Acids Res (2001) 29(22):E116;
5' nuclease assay has also been successfully used for genotyping single nucleotide polymorphisms (AYDIN A. et al. Biotechniques (2001) (4):920-2, 924, 926-8.);
Polymerase proofreading methods are used to determine SNPs identities, as described in WO 0181631;
Detection of single base pair DNA mutations by enzyme-amplified electronic transduction is described in PATOLSKY F et al. Nat Biotech. (2001) 19(3):253-257; or
Allele specific PCR methods have also been successfully used for genotyping single nucleotide polymorphisms (HAWKINS JR. et al. Hum Mutat (2002) 19(5):543-553).

Alternatively, if a patient's sequence data is already known, then obtaining may involve retrieval of the patients nucleic acid sequence data from a database, followed by determining or detecting the identity of a nucleic acid or genotype at a polymorphism site by reading the patient's nucleic acid sequence at the polymorphic site.

Once the identity of a polymorphism(s) is determined or detected an indication may be obtained as to patient outcome or prognosis based on the genotype (the nucleotide identity at a position) of the polymorphism of interest. In the present invention, polymorphisms in the PAI-1 gene, are used to obtain a prognosis or to determine patient outcome or selection of therapies. Methods for obtaining patient outcome or prognosis or for patient screening may be useful to determine the ability of a patient to recover from an inflammatory condition. Alternatively, single polymorphism sites or combined polymorphism sites may be used as an indication of a patient's ability to recover from an inflammatory condition, if they are linked to a polymorphism determined to be indicative of a patient's ability to recover from an inflammatory condition.

Once patient outcome or a prognosis is determined, such information may be of interest to physicians and surgeons to assist in deciding between potential treatment options, to help determine the degree to which patients are monitored and the frequency with which such monitoring occurs. Ultimately, treatment decisions may be made in response to factors, both specific to the patient and based on the experience of the physician or surgeon responsible for a patient's care. Treatment options that a physician or surgeon may consider in treating a patient with an inflammatory condition may include, but are not limited to the following:
(a) use of anti-inflammatory therapy;
(b) use of steroids;
(c) use of activated Protein C (drotrocogin alpha or Xigris™ from Lilly);
(d) use of modulators of the coagulation cascade (such as various versions of heparin) use of antibody to tissue factor;
(e) use of anti-thrombin or anti-thrombin III;
(f) streptokinase;
(g) use of antiplatelet agents such as clopidegrel; and
(h) Surfactant.

Alternative treatments currently in development and potentially useful in the treatment of an inflammatory condition may include, but are not limited to the following: antibodies to tumor necrosis factor (TNF) or even antibody to endotoxin (i.e. lipopolysaccharide, LPS); tumor necrosis factor receptor (TNF); tissue factor pathway inhibitors (tifacogin™ alpha from Chiron); platelet activating factor hydrolase (PAFase™ from ICOS); antibodies to IL-6; antibodies, antagonists or inhibitors to high mobility group box 1 (HMGB-1 or HMG-1 tissue plasminogen activator; bradykinin antagonists; antibody to CD-14; interleukin-10; Recombinant soluble tumor necrosis factor receptor-immunoglobulin G1(Roche); Procysteine; Elastase Inhibitor; and human recombinant interleukin 1 receptor antagonist (IL-1 RA).

Alternatively, similar methods may be employed to identify new polymorphisms in PAI-1 sequence that correlate with patient outcome or prognosis.

As described above genetic sequence information or genotype information may be obtained from a patient wherein the sequence information contains one or more single nucleotide polymorphism sites in the PAI-1 gene. Also, as previously described the sequence identity of one or more single nucleotide polymorphisms in the PAI-1 gene of one or more patients may then be detected or determined. Furthermore, patient outcome or prognosis may be assessed as described above, for example the APACHE II scoring system or the Brussels score may be used to assess patient outcome or prognosis by comparing patient scores before and after treatment. Once patient outcome or prognosis has been assessed, patient outcome or prognosis may be correlated with the sequence identity of a single nucleotide polymorphism(s). The correlation of patient outcome or prognosis may further include statistical analysis of patient outcome scores and polymorphism(s) for a number of patients.

### Clinical Phenotype

The primary outcome variable was survival to hospital discharge. Secondary outcome variables were days alive and free of cardiovascular, respiratory, renal, hepatic, hematologic, and neurologic organ system failure as well as days alive and free of SIRS (Systemic Inflammatory Response Syndrome), occurrence of sepsis, and occurrence of septic shock. SIRS was considered present when patients met at least two of four SIRS criteria. The SIRS criteria were 1) fever (>38 °C) or hypothermia (<35.5 °C), 2) tachycardia (>100 beats/min in the absence of beta blockers, 3) tachypnea (>20 breaths/min) or need for mechanical ventilation, and 4) leukocytosis (total leukocyte count > 11,000/µL) (Anonymous. Critical Care Medicine (1992) 20(6):864-74). Patients were included in this cohort on the calendar day on which the SIRS criteria were met.

A patients' baseline demographics that were recorded included age, gender, whether medical or surgical diagnosis for admission (according to APACHE III diagnostic codes (KNAUS WA et al. Chest (1991) 100(6):1619-36)), and admission APACHE II score. The following additional data were recorded for each 24 hour period (8 am to 8 am) for 28 days to evaluate organ dysfunction, SIRS, sepsis, and septic shock.

Clinically significant organ dysfunction for each organ system was defined as present during a 24 hour period if there was evidence of at least moderate organ dysfunction using the Brussels criteria (TABLE 1C) (RUSSELL JA et al. Critical Care Medicine (2000) 28(10):3405-11). Because data were not always available during each 24 hour period for each organ dysfunction variable, we used the "carry forward" assumption as defined previously (Anonymous. New England Journal of Medicine (2000) 342(18):1301-8). Briefly, for any 24 hour period in which there was no measurement of a variable, we carried forward the "present" or "absent" criteria from the previous 24 hour period. If any variable was never measured, it was assumed to be normal.

To further evaluate cardiovascular, respiratory, and renal function we also recorded, during each 24 hour period, vasopressor support, mechanical ventilation, and renal support, respectively. Vasopressor use was defined as dopamine > 5 µg/kg/min or any dose of norepinephrine, epinephrine, vasopressin, or phenylephrine. Mechanical ventilation was defined as need for intubation and positive airway pressure (i.e. T- piece and mask ventilation were not considered ventilation). Renal support was defined as hemodialysis, peritoneal dialysis, or any continuos renal support mode (e.g. continuous veno-venous hemodialysis).

To assess duration of organ dysfunction and to correct organ dysfunction scoring for deaths in the 28-day observation period, calculations were made of days alive and free of organ dysfunction (DAF) as previously reported (BERNARD GR et al. New England Journal of Medicine (1997) 336(13):912-8). Briefly, during each 24-hour period for each variable, DAF was scored as 1 if the patient was alive and free of organ dysfunction (normal or mild organ dysfunction, Table 1).
DAF was scored as 0 if the patient had organ dysfunction (moderate, severe, or extreme) or was not alive during that 24-hour period. Each of the 28 days after ICU admission was scored in each patient in this fashion. Thus, the lowest score possible for each variable was zero and the highest score possible was 28. A low score is indicative of more organ dysfunction as there would be fewer days alive and free of organ dysfunction.

Similarly, days alive and free of SIRS (DAF SIRS) were calculated. Each of the four SIRS criteria were recorded as present or absent during each 24 hour period. Presence of SIRS during each 24 hour period was defined by having at least 2 of the 4 SIRS criteria. Sepsis was defined as present during a 24 hour period by having at least two of four SIRS criteria and having a known or suspected infection during the 24 hour period (Anonymous. Critical Care Medicine (1992) 20(6):864-74). Cultures that were judged to be positive due to contamination or colonization were excluded. Septic shock was defined as presence of sepsis plus presence of hypotension (systolic blood pressure < 90 mmHg or need for vasopressor agents) during the same 24 hour period.

### Haplotypes and Selection of htSNPs

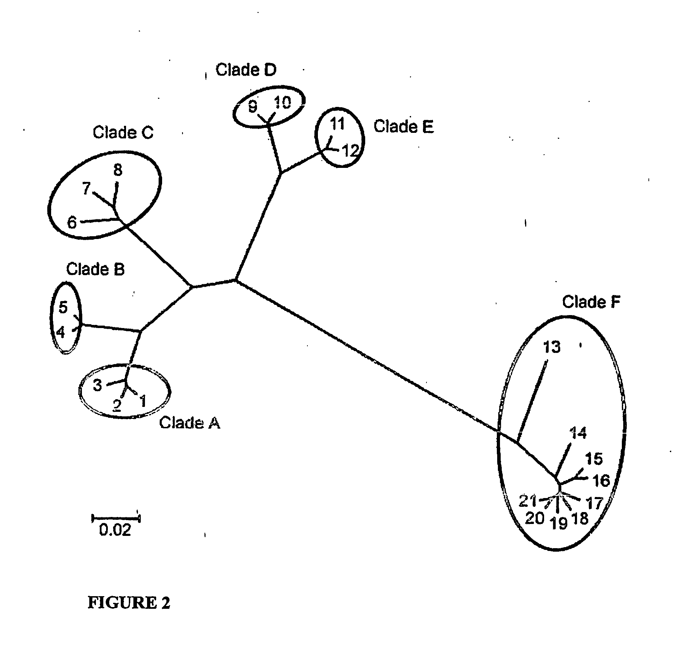

Using unphased Caucasian genotypic data (from **pga.mbt.washington.edu** (RIEDER MJ et al. ACCESSION AF386492, Vol. 2001: SeattleSNPs. NHLBI Program for Genomic Applications, UW-FHCRC, Seattle, WA (2001)) haplotypes were inferred using PHASE software (Figure 1). MEGA 2 was then used to infer a phylogenetic tree to identify major haplotype clades (Figure 2). Haplotypes were sorted according to the phylogenetic tree and haplotype structure was inspected to choose haplotype tag SNPs (htSNPs). It was found that 4 htSNPs uniquely identified the major haplotype clades (Figures 1 and 2). The 4G/5G polymorphism, which corresponds to -675 in DAWSON et al. (1993) and position 837 of SEQ ID NO:1 (NCBI ID: rs1799889) and four htSNPs: 11312 (9732) G/A, 12580 (11000) T/G; 12750 (11170) G/A; 13985 (12405) C/T) (bracketed positions numbered according to the start (1581) of the PAI-1 protein coding region - unbracketed positions numbered according of the sequence represented by accession number AF386492) were then genotyped in the patient cohort. Finally, PHASE was used to infer haplotypes from the genotyped htSNPS in each patient in the critically ill cohort with SIRS.

### Genotyping

DNA was extracted from peripheral blood samples using a QIAamp DNA Blood Maxi Kit (Qiagen). The 4G/5G polymorphism was genotyped using a restriction fragment length strategy. The htSNPs were genotyped using Masscode tagging (Qiagen Genomics, Inc) (KOKORIS M et al Molecular Diagnosis (2000) 5(4):329-40). The genotyping success rate was >99% for all SNPs.

### 3. EXAMPLES

### Patient Cohort for Examples 1 and 2

Data were collected for 235 consecutive patients who were admitted to the Intensive Care Unit (ICU) of St. Paul's Hospital. This ICU is a mixed medical - surgical ICU in a tertiary care, university-affiliated teaching hospital of the University of British Columbia. Results are reported for only the Caucasian patients (78.6% of all admissions) who were successfully genotyped (n=223) in order to decrease the potential confounding influence of population admixture secondary to ethnic diversity, on associations between genotype and phenotype. These 235 patients make up the cohort of this study.

### Example 1- Haplotype clade deduction

First haplotypes of the PAI-1 gene (Figure 1) were deduced, the major haplotype clades were identified (Figures 1 and 2), and a minimum set of htSNPs defining the major haplotype clades (Figure 1) were selected. The htSNPs and the 4G/5G polymorphism were in Hardy-Weinberg equilibrium (Table 2). Furthermore, it was found that haplotypes containing the 4G allele could be grouped into the allele that contained the minor T allele of a C/T polymorphism at position 13985 and the allele that contained the minor G allele of a T/G polymorphism at position 12580 (Figures 1 and 2). The haplotype map in Figure 1, shows PAI-1 gene haplotypes in Caucasians. Haplotypes were inferred using PHASE from available data, and represented in the style of PATIL et al. (Science (2001) 294(5547):1669-70). Columns indicate polymorphic loci and rows indicate distinct haplotypes inferred in Caucasian individuals. Light cells indicate rare alleles, and dark cells indicate common alleles. The map was generated from genotype data (available publicly at http://pga.mbt.washington.edu/) of 23 unrelated Caucasians from the Coriell registry of patients. Haplotypes were inferred using the statistical program PHASE 2.0 (STEPHENS M and DONNELLY P Am J Human Genet. (2003) 73(6):1162-1169). Polymorphisms that provide the same information as the 12580G allele were chosen based on this haplotype map. These polymorphisms provide the same information as the 12580G allele. One method is to positively identify the 12580G allele (section A) either directly or in combination. Another method is to positively identify the 12580T allele (section B), either directly or in combination, and thereby negatively identify the 12580G allele. Haplotypes within each clade are very similar, while clades differ substantially from each other. MEGA II was used to sort haplotypes into clades separated by heavier lines.

An unrooted phylogenetic tree of PAI-1 haplotype clades identified in Figure 1, is shown in Figure 2. Figure 2 was drawn using MEGA II where line length reflects relative evolutionary distance. Each clade, shown with circles, is identified by haplotype tag SNPs (htSNPs) which are representative of specific clades. Recombination of the 4G allele from its ancestral clade (htSNP allele 12580 G) appears to have occurred with an evolutionarily very distant clade (htSNP allele 13985 T).

**TABLE 2**

| **Genotype and Allele Frequencies for 4G/5G and Four htSNPS in a Cohort of Critically III Adults who had SIRS** | | | | | |
|---|---|---|---|---|---|
| | Genotype Frequencies | | | Allele Frequencies | |
| | 4G/4G | 4G/5G | 5G/5G | 4G | 5G |
| 837 (4G/5G) | 0.27 | 0.53 | 0.20 | 0.53 | 0.47 |
| | | | | | |

| | GG | GA | AA | G | A |
|---|---|---|---|---|---|
| 11312 G/A (9732) | 0.77 | 0.21 | 0.02 | 0.88 | 0.13 |
| | | | | | |

| | TT | TG | GG | T | G |
|---|---|---|---|---|---|
| 12580 T/G (11000) | 0.35 | 0.49 | 0.16 | 0.59 | 0.41 |
| | | | | | |

| | GG | GA | AA | G | A |
|---|---|---|---|---|---|
| 12750 G/A (11170) | 0.63 | 0.34 | 0.03 | 0.80 | 0.20 |
| | | | | | |

| | CC | CT | TT | C | T |
|---|---|---|---|---|---|
| 13985 C/T (12405) | 0.81 | 0.17 | 0.02 | 0.90 | 0.10 |

### Example 2 - Haplotype Clade Patient Outcome 4G Allele v. PAI-1 Clades

As shown in Figure 3, two of the six haplotype clades, D and F, are combined to make up the 4G allele and defined by: the 13985 T and the 12580 G minor alleles respectively. It was found that patients having both of the 4G haplotype clades did not demonstrate decreased hospital survival (Figure 3). In fact, the clade containing the 13985 T allele had the highest survival of all clades - in direct contrast to reporter results for the 4G allele (HERMANS (1999); MENGES (2001); and WESTENDORP (1999)). The 4G (875) allele was similarly not associated with altered survival, organ dysfunction, SIRS, sepsis, or septic shock. In contrast, those patients having 12580 G allele had markedly decreased hospital survival compared to the 13985 T allele (p<0.01) and compared to all other haplotype clades (p<0.03) (Figure 3).

### Example 3 - Patient Outcomes for G4/G5 Genotypes

When only the 4G/5G polymorphism was examined, intermediate results reflecting the addition of the increased survival of the haplotype clade, containing 13985 T allele, to the decreased survival of the haplotype clade, containing the 12580 G allele, were found. The frequency distribution of the 4G/4G, 4G/5G and 5G/5G genotypes were 26.6%, 52.1 % and 20.1 % which is similar to the frequencies reported in other critically ill Caucasians (MENGES T et al. Lancet (2001) 357(9262):1096-7). Hospital survival rates were 54% for 4G/4G, 61% for 4G/5G, and 74% for 5G/5G groups, which were not statistically significantly different (p = 0.09). However, the 4G allele remained associated with significantly fewer days alive and free of respiratory dysfunction (p< 0.05), hepatic dysfunction (p< 0.05), and neurologic dysfunction (p<0.05) (TABLE 3). Cardiovascular, renal, and coagulation DAF scores were not significantly different between 4G/5G genotypes (Table 3). These results were supported by the findings of no difference in DAF vasopressors (additional measurement of cardiovascular function) but significantly fewer DAF ventilator (additional measurement of respiratory function) in patients who had the 4G allele (Table 3). Those patients who had the 4G allele were found to have a significantly increased duration of SIRS (DAF SIRS: 19.8 out of 28 for 4G/4G, 20.8 for 4G/5G, 23.4 for 5G/5G), a trend to increased occurrence of sepsis (94% for 4G/4G, 99% for 4G/5G, 74% for 5G/5G, p=0.08) and a significant increase in occurrence of septic shock (94% for 4G/4G, 99% for 4G/5G, 74% for 5G/5G, p=0.04) during the 28 day study period.

**TABLE 3**

| **Days Alive and Free of Organ Failure (out of a possible 28) for PAI-1 4G/5G Polymorphism Genotypes in a Cohort of Critically III Adults who had SIRS** | | | | |
|---|---|---|---|---|
| | 4G/4G | 4G/5G | 5G/5G | p |
| Cardiovascular | 18.5±2.6 | 19.7±1.8 | 22.3±2.7 | 0.07 |
| Vasopressors (marker of Cardiovascular dysfunction) | 17.2±2.6 | 18.6±1.9 | 21.0±2.8 | 0.08 |
| Respiratory | 7.2±2.1 | 5.9±1.4 | 12.3±2.9 | 0.02 |
| Ventilator (marker of Respiratory dysfunction) | 12.7±2.8 | 13.9±1.9 | 17.0±2.9 | 0.04 |
| Renal | 16.5±2.7 | 16.3±2.0 | 18.0±3.3 | 0.52 |
| Renal Support (marker of Renal dysfunction) | 18.7±2.7 | 18.1±2.0 | 19.4±3.2 | 0.85 |
| Hepatic | 18.0±2.8 | 19.0±1.9 | 23.0±2.8 | 0.02 |
| Hematologic | | | | |
| Neurologic | 18.4±2.6 | 19.5±1.9 | 22.8±2.6 | 0.04 |

| | | | | |
|---|---|---|---|---|
| Mean ± 95% Confidence Interval | | | | |

### Example 4 - Patient Outcomes for PAI-1 12580 Genotypes v. 4G/5G Genotypes

To assess duration of organ dysfunction and to correct organ dysfunction scoring for deaths in the 28-day observation period, days alive and free of organ dysfunction (DAF) were calculated. During each 24-hour period for each variable, DAF was scored as "1" if the patient was alive and free of organ dysfunction (normal or mild organ dysfunction- TABLE 1C). DAF was scored as "0" if the patient had organ dysfunction (moderate, severe, or extreme - TABLE 1 C) or was not alive during that 24-hour period. Each of the 28 days after ICU admission was scored in each patient in this fashion. Thus, the lowest score possible for each variable was zero and the highest score possible was 28. A low score is indicative of more organ dysfunction as there would be fewer days alive and free of organ dysfunction. There were no significant differences for the PAI-1 12580 genotypes between groups (either GG/GT vs TT or GG, GT and TT separately as shown in TABLES 4A and 4B below) with respect to age, sex, APACHE II- score and percent of patients admitted with surgical diagnosis. Similary, there were no significant differences for the PAI-1 4G/5G genotypes between groups (either 5G/5G vs 5G/4G and 4G/4G or any 4G separately as shown in TABLES 5A and 5B below).

**Table 4A. Baseline statistics of 587 Caucasian patients with SIRS grouped by PAI-1 12580 genotype. No significant differences in age, sex, APACHE II score or surgical diagnosis on admit were observed between PAI-1 12580 genotype groups (GG, GT or TT).**

| | Age | % Female | APACHE II Score | % Surgical Diagnosis |
|---|---|---|---|---|
| GG | 58±2 | 38 | 24 ± 1 | 27 |
| GT | 57 ± 1 | 35 | 23 ±1 | 20 |
| TT | 57 ± 1 | 41 | 23 ± 1 | 28 |
| p Value | NS | NS | NS | NS |

**Table 4B. Baseline statistics of 587 Caucasians with SIRS grouped by PAI-1 12580 genotype (GG/GT vs TT). No significant differences in age, sex, APACHE II score or surgical diagnosis on admit were observed between groups.**

| | Age | % Female | APACHE II Score | % Surgical Diagnosis |
|---|---|---|---|---|
| GG/GT | 58 ± 1 | 36 | 23 ± 1 | 22 |
| TT | 57 ± 1 | 41 | 23 ± 1 | 28 |
| p Value | NS | NS | NS | NS |

**Table 5A. Baseline statistics of 314 Caucasians with SIRS grouped by PAI-1 4G/5G (837) genotype (5G/5G, 5G/4G and 4G/4G). No significant differences in baseline statistics were observed.**

| | Age | % Female | APACHE II Score | % Surgical Diagnosis |
|---|---|---|---|---|
| 5G/5G | 59 ± 2 | 42 | 22 ± 1 | 29 |
| 5G/4G | 57 ± 1 | 34 | 22 ± 1 | 26 |
| 4G/4G | 61 ± 2 | 33 | 21 ± 1 | 37 |
| p Value | NS | NS | NS | NS |

**Table 5B. Baseline statistics of 314 Caucasians with SIRS grouped by PAI-1 4G/5G (837) allele (any 4G vs 5G/5G). No significant differences were observed in baseline statistics.**

| | Age | % Female | APACHE II Score | % Surgical Diagnosis |
|---|---|---|---|---|
| Any 4G | 58 ± 1 | 34 | 22 ± 1 | 29 |
| 5G/5G | 59 ± 2 | 42 | 22 ± 1 | 29 |
| p Value | NS | NS | NS | NS |

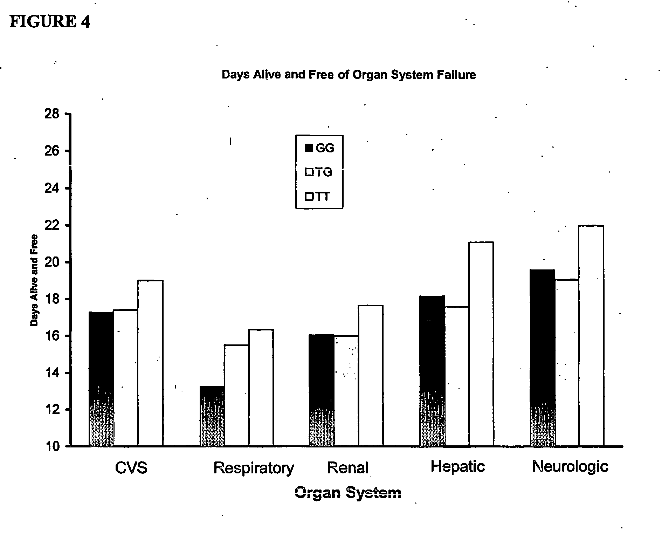

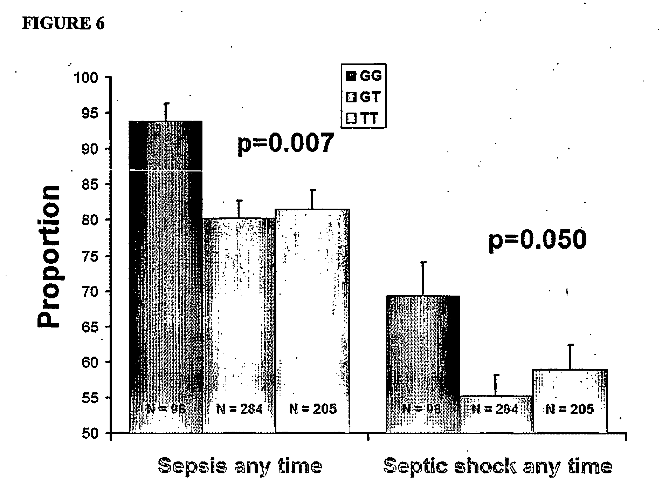

Patients having haplotypes containing the 12580 G allele had marked organ dysfunction of respiratory (p<0.05), renal (p<0.05), hepatic (p<0.05), and neurologic (p<0.05) organ systems over the 28 day study period (Figure 4). Days alive and free of all organ dysfunction for 12580 GG was 1.8, GT 4.6, and TT 8.2 (p<0.01). Thus, the 12580 G allele was associated with markedly worse overall organ failure. In addition, the 12580 G allele was associated with more SIRS (p<0.05), more sepsis (p<0.05), and more septic shock (p<0.05) (Figures 5 and 6). Thus, the 12580 G allele was associated with markedly adverse clinical phenotype expressed as decreased survival, worse organ dysfunction, and worse SIRS.

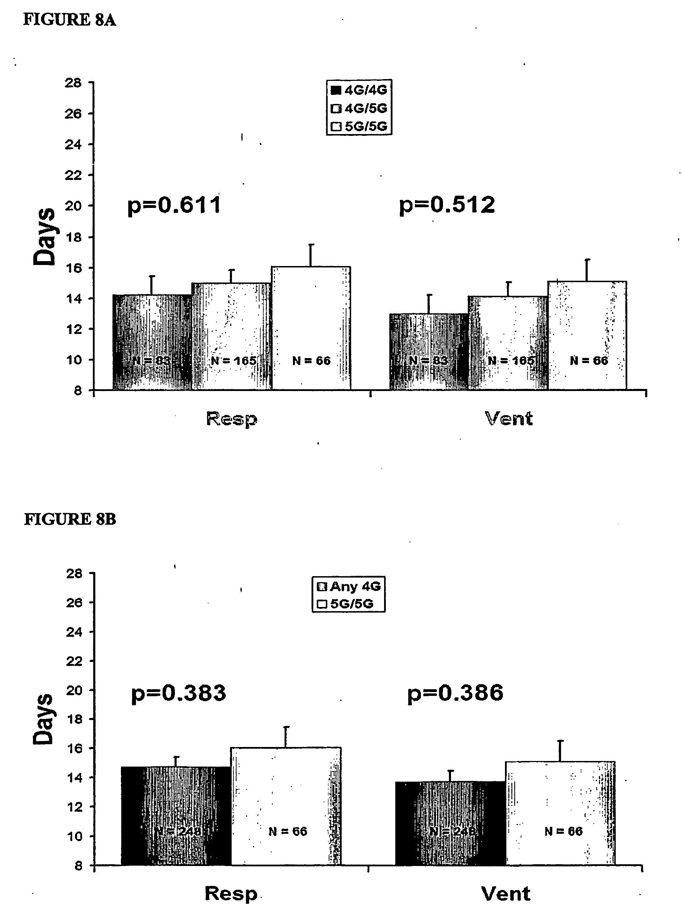

It was found that there was no statistically significant difference or trend in DAF when grouped by PAI-1 4G/5G (837) genotype (Figures 8 A, 8B, 10 A, 10 B, 12 A, and 12 B). However, when patients were grouped by PAI-1 12580 genotype distinct trends in DAF were associated with increased numbers of the 12580G allele.

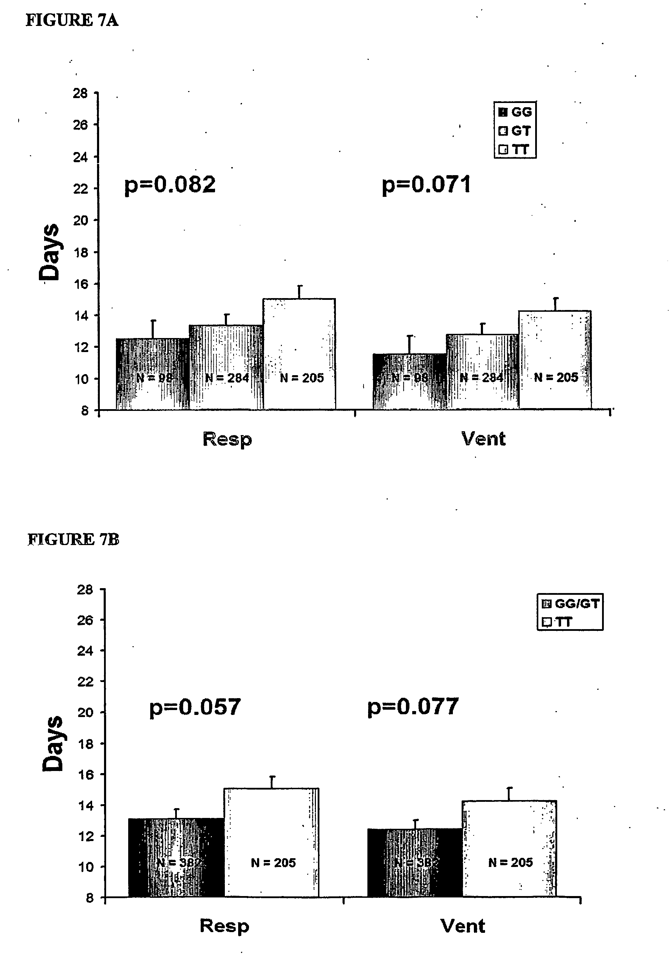

In Figure 7A, days alive and free of respiratory dysfunction and mechanical ventilation by PAI-1 12580 genotype (GG, GT and TT separately) in 587 critically ill Caucasians with the systemic inflammatory response syndrome (SIRS) is shown. Those patients possessing the 12580G allele had a strong trend toward fewer days alive and free of respiratory dysfunction and mechanical ventilation based on the number of copies of the 12580G allele they had. Those patients with 2 copies of the 12580G allele (N = 98) had the fewest days alive and free of respiratory dysfunction (13 days) and mechanical ventilation (12 days). Patients with only one copy of the 12580G allele (N =284) had intermediate values for days alive and free of respiratory dysfunction (13 days) and mechanical ventilation (13 days). Patients with 0 copies of the 12580 G allele (i.e. TT homozygotes; N = 205) had the greatest number of days alive and free of respiratory dysfunction (15 days) and mechanical ventilation (14 days).

Similarly, Figure 7B, shows days alive and free of respiratory dysfunction and mechanical ventilation by PAI-1 12580 genotype (GG/GT vs TT) in 587 critically ill Caucasians with SIRS. Those patients with either 1 or 2 copies of the 12580G allele (GG or GT, N = 382) had a strong trend toward fewer days alive and free of respiratory dysfunction (p=0.057) and mechanical ventilation (p=0.077) compared to those patients having 0 copies of the G allele (i.e. 12580 TT homozygotes, N = 205).

Figures 8A and 8B show days alive and free of respiratory dysfunction and mechanical ventilation by PAI-1 4G/5G (837) genotype (showing 4G/4G, 4G/5G and 5G/5G separately and any 4G vs 5G/5G) in 314 critically ill Caucasians with the systemic inflammatory response syndrome (SIRS). No significant differences or trends in DAF respiratory dysfunction or mechanical ventilation were observed based on PAI-1 4G/5G genotype.

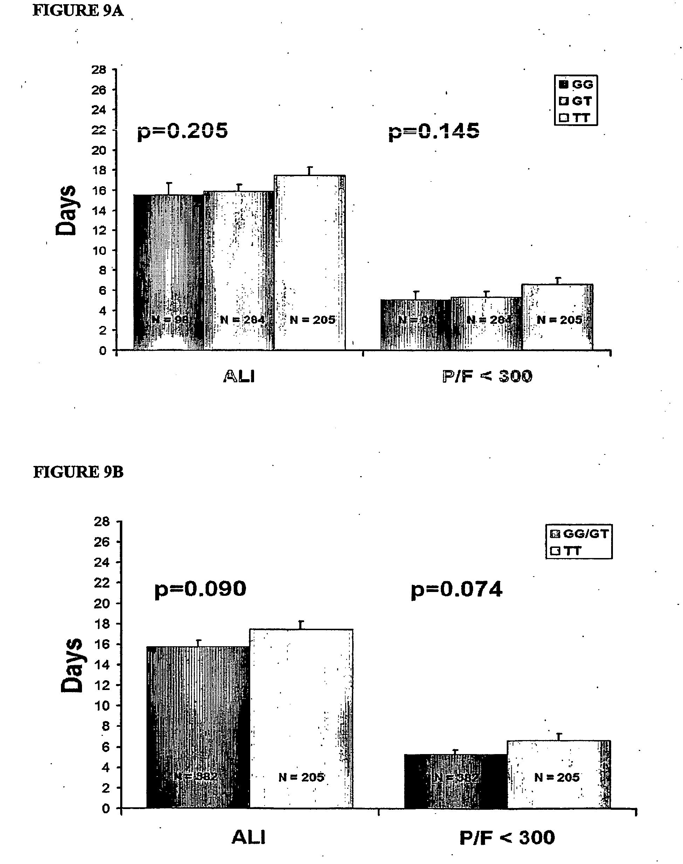

In Figure 9A, days alive and free of acute lung injury (ALI) and PₐO₂/FᵢO₂ (P/F) ratio greater than 300 mmHg by PAI-1 12580 genotype (GG, GT and TT separately) in 587 critically ill Caucasians with SIRS are shown. Those patients having the 12580G allele had a trend toward fewer days alive and free of ALI and P/F < 300 based on the number of copies of the 12580G allele they had. Patients with 2 copies of the 12580G allele (N = 98) had the fewest days alive and free of ALI (16 days) and P/F < 300 (5 days). Patients with 1 copy of the 12580G allele (N = 284) had intermediate values for, days alive and free of ALI (16 days) and P/F < 300 (5 days). Patients with 0 copies of the 12580G allele had the greatest number of days alive and free of ALI (17 days) and P/F < 300 (7 days).

Similarly, Figure 9B, shows days alive and free of acute lung injury and PₐO₂/FᵢO₂ < 300 mmHg by PAI-1 12580 genotype (GG/GT vs TT) for 587 Caucasians with SIRS. Those patients with either 1 or 2 copies of the 12580G allele (GG or GT, N = 382) had a trend toward fewer days alive and free of acute lung injury (16 days) and PaO2/FiO2 < 300 mmHg (5 days) compared to those patients with 0 copies of the 12580G allele (17 and 7 days, resp; N = 205).

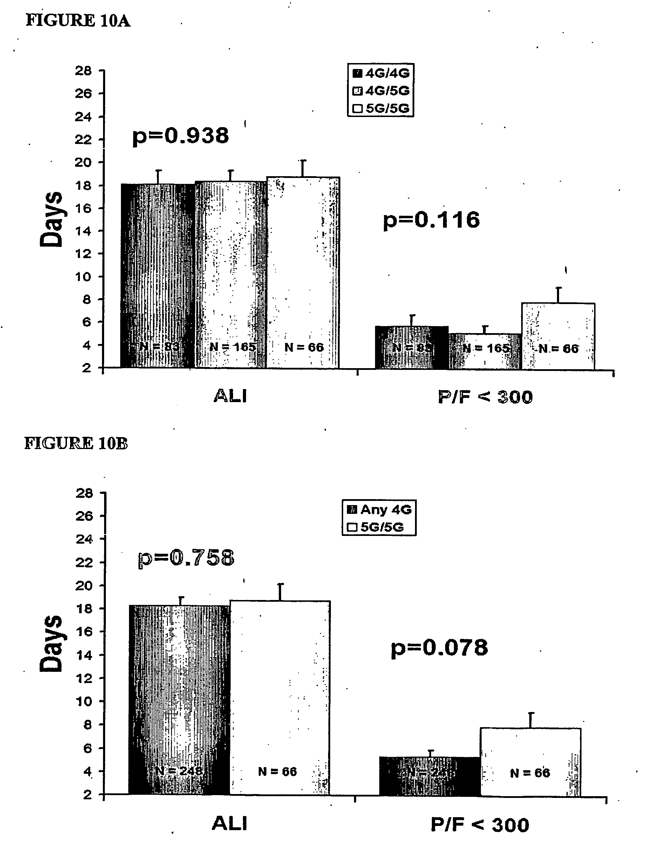

Figure 10A shows days alive and free of acute lung injury (ALI) and PₐO₂/FᵢO₂ (P/F) ratio greater than 300 mmHg by PAI-1 4G/5G/ (837) genotype (showing 4G/4G, 4G/5G and 5G/5G separately) in 314 critically ill Caucasians with SIRS. No significant differences or trends in DAF ALI or P/F < 300 were observed based on PAI-1 4G/5G genotype. Similarly, Figure 10B shows days alive and free of acute lung injury and PₐO₂/FᵢO₂ < 300 mmHg by PAI-1 4G/5G allele (any 4G vs 5G/5G) in 314 critically ill Caucasians with SIRS. No significant difference or trend in DAF ALI was observed based upon PAI-1 4G/5G allele. A trend toward fewer DAF P/F < 300 in the group "any 4G" was observed, but this trend was not as significant as that observed based on the PAI-112580 allele (Figure 9B).

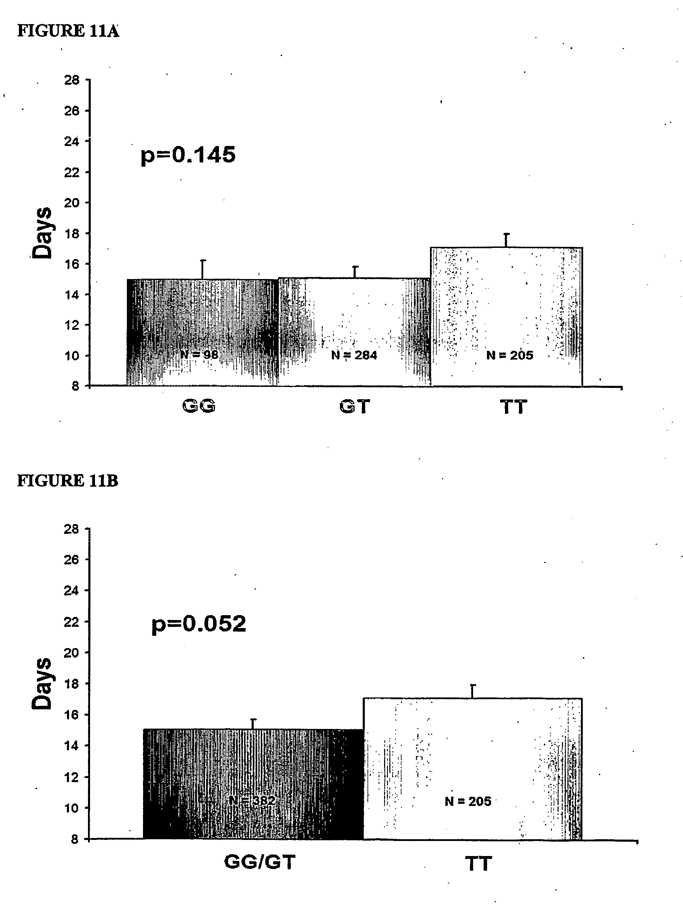

In Figure 11A, days alive and free of steroid use by PAI-1 12580 genotype in 587 critically ill Caucasians with SIRS is shown. Patients with 2 copies of the 12580G allele (N = 98) had the fewest days alive and free of steroid use (15 days). Patients with 1 copy of the 12580G allele (N = 284) had similar values for days alive and free of steroid use (15 days). Patients with 0 copies of the 12580G allele (N = 205) had the greatest number of days alive and free of steroid use (17 days). Similarly, Figure 11B, shows days alive and free of steroid use by PAI-1 12580 genotype (GG/GT vs TT) for 587 critically ill Caucasians with SIRS. Those patients having either 1 or 2 copies of the 12580G allele (i.e. GG or GT; N = 382) had a strong trend toward fewer days alive and free of steroid use (15 days) compared to those patients with 0 copies (17 days; N = 205).

Figure 12A show days alive and free of steroid use by PAI-1 4G/5G (837) genotype in critically ill Caucasians with SIRS (grouped 4G/4G, 4G/5G and 5G/5G separately). No significant difference or trend in DAF steroid use was observed based on the PAI-1 4G/5G genoytpe.
Similarly, Figure 12B shows days alive and free of steroid use by PAI-1 4G/5G (837) were the same patients in 12A are grouped as any 4G vs 5G/5G. Again, no significant difference or trend was observed in DAF steroid use based on PAI-1 4G/5G allele.

### Example 5 - Patient Outcomes for PAI-1 12580 Genotypes v. 4G/5G Genotypes in Surgery Patients

Plasminogen activator inhibitor-1 (PAI-1) levels were measured in 67 Caucasian cardiovascular surgery patients who had cardiopulmonary bypass. Serum levels were measured by enzyme-linked immunosorbent assay (ELISA) (Immulyse PAI-1, BioPool International) and presented based on PAI-1 12580 genotype (Figures 13A/B, 15A/B, 16 and 17A/B). No significant differences were observed between PAI-1 12580 genotype groups based on age or sex (as shown in TABLES 6A and 6B below).

**Table 6A. Baseline statistics for 67 Caucasians who underwent on-pump cardiopulmonary bypass surgery and had PAI-1 serum levels measured. No significant differences in age or sex were observed between PAI-1 12580 genotype groups (GG, GT and TT).**

| | Age | % Female |
|---|---|---|
| GG | 74 ± 5 | 60 |
| GT | 62 ± 2 | 27 |
| TT | 68 ± 2 | 33 |
| p Value | NS | NS |

**Table 6B. Baseline statistics for 67 Caucasians who underwent on-pump cardiopulmonary bypass surgery and had PAI-1 serum levels measured. No significant differences in age or sex were observed between PAI-1 12580 genotype groups (GG/GT vs TT).**

| | Age | % Female |
|---|---|---|
| GG/GT | 64 ± 2 | 31 |
| TT | 68 ± 2 | 33 |
| P Value | NS | NS |

Similarly, PAI-1 levels were also measured in 55 Caucasian cardiovascular surgery patients who had cardiopulmonary bypass and for comparison purposes serum levels were presented based on PAI-1 4G/5G (837) genotype (Figures 14A/B and 18A/B). A significant difference in proportion of female patients was observed (shown in TABLES 7A and 7B below), and this was accounted for in later analyses_{.}

**Table 7A. Baseline statistics for 55 Caucasians who underwent on-pump cardiopulmonary bypass surgery and had PAI-1 serum levels measured. No significant difference in age was observed between PAI-1 4G/5G groups (4G/4G, 4G/5G and 5G/5G). A significant difference in proportion of female patients was observed, and this was accounted for in later analyses.**

| | Age | % Female |
|---|---|---|
| 4G/4G | 68 ± 2 | 53 |
| 4G/5G | 64 ± 2 | 15 |
| 5G/5G | 68 ± 2 | 36 |
| p Value | NS | 0.037 |

**Table 7B. Baseline statistics for 55 Caucasians who underwent on-pump cardiopulmonary bypass surgery and had PAI-1 serum levels measured. No significant differences in age or sex were observed between PAI-1 4G/5G genotype groups (any 4G vs 5G/5G).**

| | Age | % Female |
|---|---|---|
| Any 4G | 66 ± 2 | 29 |
| 5G/5G | 68 ± 2 | 36 |
| p Value | NS | NS |

Surprisingly, it was found that there was no statistically significant difference (or even a trend) in PAI-1 serum levels and systemic vascular resistance index (SVRI) in patents 4 hours post-operatively after cardiopulmonary bypass surgery when grouped by PAI-1 4G/5G (837) genotype. However, when patients were grouped by PAI-1 12580 genotype a statistically significant decrease in PAI-1 serum levels was associated with increased numbers of the 12580G allele, both post and pre operatively. Similarly, the PAI-I 12580 genotype showed a statistically significant decrease in SVRI associated with the 12580G allele post-operatively. Thus, the PAI-1 12580 G allele was associated with more vasodilation (as shown by lower SVRI), and lower PAI-1 levels after cardiac surgery than PAI-1 12580 T. This represents independent confirmation in an independent cohort of patients who had cardiac surgery of the findings of PAI-1 12580 G in the ICU cohort in that PAI-1 12580 G was associated with worse survival and organ dysfunction in the ICU cohort and lower SVRI and lower PAI-1 levels in the cardiac surgery cohort.

In Figure 13A, mean pre-operative PAI-1 serum levels in 24 Caucasians who underwent on-pump cardiopulmonary bypass surgery grouped by PAI-1 12580 genotype (GG, GT and TT separately) are shown. The 12580 TT (N = 3) homozygotes had a mean pre-operative PAI-1 serum level of 146.9 ng/mL, the GT heterozygotes (N = 14) had a mean pre-operative PAI-1 serum level of 102.2 ng/mL, and the GG homozygotes (N = 7) had a mean pre-operative serum PAI-1 level of 84.5 ng/mL. A statistically significant trend toward lower serum PAI-1 levels with increasing copy number of the 12580G allele is present (p=0.044). Statistics were performed on log transformed data since levels were not normally distributed.

In Figure 13B, mean pre-operative serum PAI-1 levels in 24 Caucasians who underwent on-pump cardiopulmonary bypass surgery grouped by PAI-1 12580 genotype (GG/GT vs IT) are shown. Here as before the TT homozygotes (N = 3) had higher mean pre-operative serum PAI-1 levels of 154.4 ng/mL compared to those patients with either the GG or GT genotypes (N = 21, mean serum PAI-1 level of 94.5 ng/mL). Statistical significance is not reached (p=0.077), but a value indicating a trend is observed. Statistics were performed on log transformed data. In Figure 14A, serum PAI-1 levels measured 4 hours post-operatively in 55 Caucasians patients who underwent cardiopulmonary bypass surgery are shown. No significant difference was observed in serum PAI-1 level based on 4G/5G (837) genotype, and neither was there any trend in PAI-1 levels based on these genotypes. The difference in sex was included in a linear regression model and had no effect on lack of statistical significance. Similarly, Figure 14B shows serum PAI-1 levels measured 4 hours post-operatively in 55 Caucasians who underwent on-pump cardiopulmonary bypass surgery by any 4G vs. 5G/5G. Again, no significant difference in serum PAI-1 levels was observed based on 4G/5G (837) allele (any 4G vs 5G/5G), and neither was any significant trend observed. Statistics were performed on log-transformed data.

In Figures 15A and 15B, mean serum PAI-1 levels of 67 Caucasians who underwent on-pump cardiopulmonary bypass surgery collected at 4 hours post surgery grouped by PAI-1 12580 genotype (TT, GT and GG separately and GG/GT vs TT) are shown. This group includes as a subset those patients (N = 24) for whom data was collected at the pre-operative time-point, and therefore represents a confirmation of the trends observed in the aforementioned group (i.e. 12580G allele associated with lower serum PAI-1 levels) in a group with a larger sample size. The 12580 TT homozygotes (N = 21) had the highest mean serum PAI-1 levels at 195.1 ng/mL, followed by the GT heterozygotes with 130.3 ng/mL and the GG homozygotes with 111.3 ng/mL. In Figure 15B, the 12580 TT homozygotes had statistically significantly higher mean serum PAI-1 levels post-operatively (195.1 ng/mL) compared to those patients with either the GT or GG genotypes (126.0; p=0.042). A clear trend once again emerges wherein lower serum PAI-1 levels are associated with increasing numbers of the 12580G allele. Statistics were performed on log transformed data.

In Figure 16 mean differences in serum PAI-1 levels from pre-operative to 4 hours post-operative in 24 Caucasians who underwent on-pump cardiopulmonary bypass surgery is shown. Those patients who were homozygous for the 12580T allele had the greatest increase in PAI-1 levels pre-operative to post-operative (208.0 ng/mL). Heterozygous patients had an intermediate increase in PAI-1 levels (158.9 ng/mL), and those patients who were homozygous for the 12580G allele had the lowest mean increase in serum PAI-1 levels pre-operative to post-operative (43.6 ng/mL). Although statistical significance was not achieved, a clear trend is visible indicating a smaller increase in PAI-1 levels following cardiopulmonary bypass surgery with increasing copy number of the PAI-1 12580 G allele.

In Figure 17A, systemic vascular resistance index (SVRI) is shown 4 hours post-operatively in 66 Caucasians undergoing on-pump cardiopulmonary bypass surgery grouped by PAI-1 12580 genotype (GG, GT and TT separately). SVRI is calculated as SVRI = (mean arterial pressure - central venous pressure) / cardiac index, and expressed as dynes/s•cm⁻⁵. The PAI-1 12580 G allele is associated with a statistically significant trend toward lower mean SVRI 4 hours post-operatively based on the allele copy number: GG homozygotes have the lowest SVRI values (1493.9 ±144.8 dynes/s•cm⁻⁵), GT heterozygotes have intermediate SVRI values (1629.7 ± 61.5 dynes/s•cm⁻⁵), and TT homozygotes have the highest SVRI values (1780.4 ± 64.6 dynes/s•cm⁻⁵). This trend is significant at the a level 0.05 (p=0.028).

Similarly, in Figure 17B, systemic vascular resistance index (SVRI) is shown 4 hours post-operatively in 66 Caucasians undergoing on-pump cardiopulmonary bypass surgery grouped by PAI-1 12580 genotype (GG/GT vs TT). SVRI was calculated as described above for Figure 17A. The PAI-1 12580 G allele (here as GG homozygotes or GT heterozygotes) is shown to be significantly associated with lower mean SVRI 4 hours post-operatively than the T allele (1604.4 ± 57.8 dynes/s•cm⁻⁵ vs 1780.4 ± 64.6 dynes/s∘cm⁻⁵ respectively; p = 0.050).

Figure 18A shows the systemic vascular resistance index (SVRI) 4 hours post-operatively in 364 Caucasians undergoing on-pump cardiopulmonary bypass surgery grouped by PAI-1 4G/5G (837) genotype (4G/4G, 4G/5G and 5G/5G separately). SVRI is calculated as SVRI = (mean arterial pressure - central venous pressure) / cardiac index, and expressed as dynes/s•cm⁻⁵. No significant difference or trend in 4 hour post-operative SVRI was observed based upon the PAI-1 4G/5G genotype. Similarly, in Figure 18B in which patients were grouped by PAI-1 4G/5G (837) allele (any 4G vs 5G/5G). SVRI is calculated as above for 18A and again no significant difference or trend in 4 hour post-operative SVRI was observed based on the PAI-1 4G/5G allele.

### Statistical Analysis

We compared measures of disease severity using dominant and co-dominant models. We tested for differences between genotype groups using ANOVA for continuous data and a Fisher exact test for discrete data. When appropriate Student's t-test and linear regression were also used.

The sample size described herein (260), compares with sample sizes in trauma of 61 (MENGES T et al. Lancet (2001) 357(9262):1096-7), in pediatric meningococcemia of 175 (DAWSON et al. (1993)), and adult meningococcal septic shock of 50 (WESTENDORP RG et al. Lancet (1999) 354(9178):561-3). Another possible source of error in association studies is population admixture (CARDON LR and BELL JI Nature Reviews Genetics (2001) 2:91-9). This issue was addressed by limiting the analysis to Caucasians and also because this group comprised the majority of our patients available for study.

### SUMMARY

The PAI-1 12580 G allele is associated with greater evidence of SIRS and severe cardiovascular and respiratory dysfunction in a critically ill SIRS population and in a post CPB SIRS population. The critically ill SIRS population demonstrates that severe SIRS in the patients with the GG genotype was associated with more severe SIRS and more cardiovascular and respiratory failure (including more acute lung injury, more use of vasopressors, more use of steroids), but also in trends to additional organ system dysfunction and importantly, to decreased survival: These observations were confirmed in an analogous but completely independent SIRS population of critically ill patients who had CPB. In the CPB population SIRS was induced by cardiac surgery and the cardiopulmonary bypass procedure itself without evidence of infection. Evidence for increased SIRS in those patients having the GG genotype in this population is provided by the observation of greater reduction in systemic vascular resistance index (SVRI).

Critically ill patients with the 12580 G allele had significantly worse outcomes as indicated by lower survival, more SIRS, more severe cardiovascular and respiratory failure, and strong trends to more severe hepatic renal, neurologic, and coagulation dysfunction. The poor clinical phenotype of the patients who had the 12580 G allele was also associated with greater use of corticosteroids. It is suspected that the reason for increased use of corticosteroids (see Figures 11A and B), is that the clinicians judged that there was a greater need for steroid treatment for severe shock and possibly prolonged respiratory failure.

Described herein are haplotypes that are novel in their ability to predict patient outcome due to inflammation, and haplotypes that are much more functional in describing patient risk than the previously described 4G/5G polymorphism. As a result, provided herein is a better strategy of predicting which patients are at greatest risk of an adverse outcome.

The PAI-1 4G allele is shown here to be divided into two distinct haplotype clades as a result of a remote recombination event. Inspection indicates that a recombinant event occurred between the 4G/5G site (position 837) in the promoter region and position 4588 (see Figure 1). In essence, a "natural experiment" occurred in which the promoter region containing the 4G allele in question, was recombined with a completely different haplotype clade (Figure 2). *Pan troglodytes* (chimpanzee) genotyping (Figure 2) suggests that 4G originated with the ancestral clade F (defined by the 12580 G allele) and recombined with the evolutionarily most distant haplotype clade D (defined by the 13985 T allele) of the PAI-1 gene (Figure 2). This "natural experimental design" therefore directly tests the hypothesis that the promoter region containing 4G is causal because the 4G allele is distributed into two different haplotype clades that can then be compared. It was found that the two 4G haplotype clades (F and D) were associated with very different outcomes. The 12580 G haplotype clade was associated with significantly reduced hospital survival and organ dysfunction while the 13985 haplotype clade was not. Thus, this "natural experiment" has lead to the conclusion that 4G is not associated with patient outcome or not strongly so.

From our results the 4G allele of PAI-1 is not associated with poor prognosis. As described herein a SNP within the 12580 G haplotype clade (clade F) is a better candidate for predicting patient outcome, because the 12580 G haplotype clade was strongly associated with adverse outcomes. In the haplotype map (Figure 1), the PAI-1 4G allele (837-) and 12580 G allele are linked by some measure of linkage disequilibrium, though not complete LD (estimated to be about r² ≈ 0.2 calculated using the Visual Genotype2 program available at http://droog.gs.washington.edu/pga/VG2.html). As a result the 12580G allele is proposed herein as a better predictor of patient outcome than the 4G allele.

In a cohort of critically ill patients, 95% having SIRS, analysis based on the haplotype structure of the PAI-1 gene suggested that the 4G/5G polymorphism was not associated with adverse outcome. Rather, a polymorphism contained within the haplotype clade defined by the minor G allele of the 12580 T/G polymorphism, was a better candidate. The 12580 G haplotype clade was associated with decreased survival and increased organ dysfunction, severity of SIRS, and occurrence of sepsis and septic shock. The association of the 12580 G haplotype clade with adverse outcome is important because it shows that the very commonly reported 4G/5G SNP is not the best predictor of patient outcome. This has implications for critically ill patients who have SIRS and possibly for patients having other diseases (e.g. stroke, myocardial infarction) in which 4G/5G has been reported as associated with adverse outcomes.

The PAI-1 12580 G allele was associated with lower serum PAI-1 levels (Figures 13A/B and 15A/B) and with fewer days alive and free of organ dysfunction (respiratory, acute lung injury), support (mechanical ventilation and steroid use) and is associated with significantly lower systemic vascular resistance index in cardiac surgical patients. Therefore, the 12580 G allele is a novel indicator of poor outcome (survival and days alive and free of organ dysfunction as described above) and lower serum PAI-1 levels.

In conclusion, the 4G/5G polymorphism of PAI-1 is not as good a predictor of outcome in patients with SIRS, but rather, a polymorphism contained within the haplotype clade defined by the minor G allele of the 12580 T/G polymorphism, is a better candidate.

## Claims

1. A method for obtaining a prognosis for a patient having or at risk of developing an inflammatory condition, the method comprising determining a genotype for one or more polymorphism sites in the plasminogen-activator-inhibitor-1 (PAI-1) gene for the patient, wherein said genotype is indicative of an ability of the patient to recover from an inflammatory condition, provided that the one or more polymorphism sites is not solely a polymorphism at a site corresponding to position 837 of SEQ ID NO.: 1.

2. The method of claim 1, wherein the one or more polymorphism sites includes position 12580 of SEQ ID NO.: 1.

3. The method of claim 1, wherein the one or more polymorphism sites are selected from positions 5645, 7121, 7437, 8070, 8406, 9463, 9466, 12219, 12580, 13889 and 14440 in SEQ ID NO: 1.

4. The method of claim 1, wherein the genotype comprises a combination of two or more polymorphism sites, the combination being selected from the group of positions corresponding to SEQ ID NO:1 consisting of:
664 A and 2037 T;
664 A and 2362 -;
664 A and 2852 A;
664 A and 5834 A;
837- and 2037 T;
837 - and 2362 -;
837 - and 2852 A;
837 - and 5834 A;
| | | | | | |
|---|---|---|---|---|---|
| one of | 5878 G | and one of | 7365 T | and one of | 4588 T |
| | 7343 G | | 7729 + | | 5404 G |
| | 13605 A | | 7771 A | | 5686 A |
| | | | 12750 A | | 5984 A |
| | | | | | 11312 A; |
and
| | | | | | | |
|---|---|---|---|---|---|---|
| one of | 2846 A | and 10381 T | and one of | 7365 T | and one of | 4588 T |
| | 6821 T | and 10381 T | | 7729 + | | 5404 G |
| | 9759 G | and 10381 T | | 7771 A | | 5686 A |
| | | | | 12750 A | | 5984 A |
| | | | | | | 11312 A. |

5. The method of any one of claims 1-4, further comprising comparing the genotype so determined with known genotypes, which are indicative of a prognosis for recovery from the same inflammatory condition as for the patient or another inflammatory condition.

6. The method of claim 1 to 5, wherein said determining of genotype is performed on a nucleic acid sample from the patient.

7. The method any one of claims 1-6, wherein said determining of genotype comprises one or more of:
(a) restriction fragment length analysis;
(b) sequencing;
(c) hybridization;
(d) oligonucleotide ligation assay;
(e) ligation rolling circle amplification;
(f) 5' nuclease assay;
(g) polymerase proofreading methods;
(h) allele specific PCR; and
(i) reading sequence data.

8. The method of any one of claims 1-7, wherein the genotype of the patient is indicative of a decreased likelihood of recovery from an inflammatory condition.

9. The method of claim 8, wherein the prognosis is indicative of severe cardiovascular or respiratory dysfunction in critically ill patients.

10. The method of any one of claims 1 to 7, wherein the genotype of the patient is indicative of a increased likelihood of recovery from an inflammatory condition.

11. The method of claim 10, wherein the prognosis is indicative of less severe cardiovascular or respiratory dysfunction in critically ill patients.

12. The method of any one of claims 1 to 11, wherein the inflammatory condition is selected from the group consisting of: sepsis, septicemia, pneumonia, septic shock, systemic inflammatory response syndrome (SIRS), Acute Respiratory Distress Syndrome (ARDS), acute lung injury, infection, pancreatitis, bacteremia, peritonitis, abdominal abscess, inflammation due to trauma, inflammation due to surgery, chronic inflammatory disease, ischemia, ischemia-reperfusion injury of an organ or tissue, tissue damage due to disease, tissue damage due to chemotherapy or radiotherapy, and reactions to ingested, inhaled, infused, injected, or delivered substances, glomerulonephritis, bowel infection, opportunistic infections, and for patients undergoing major surgery or dialysis, patients who are immunocompromised, patients on immunosuppressive agents, patients with HIV/AIDS, patients with suspected endocarditis, patients with fever, patients with fever of unknown origin, patients with cystic fibrosis, patients with diabetes mellitus, patients with chronic renal failure, patients with bronchiectasis, patients with chronic obstructive lung disease, chronic bronchitis, emphysema, or asthma, patients with febrile neutropenia, patients with meningitis, patients with septic arthritis, patients with urinary tract infection, patients with necrotizing fasciitis, patients with other suspected Group A streptococcus infection, patients who have had a splenectomy, patients with recurrent or suspected enterococcus infection, other medical and surgical conditions associated with increased risk of infection, Gram positive sepsis, Gram negative sepsis, culture negative sepsis, fungal sepsis, meningococcemia, post-pump syndrome, cardiac stun syndrome, myocardial infarction, stroke, congestive heart failure, hepatitis, epiglotittis, E. coli 0157:H7, malaria, gas gangrene, toxic shock syndrome, mycobacterial tuberculosis, Pneumocystic carinii, pneumonia, Leishmaniasis, hemolytic uremic syndrome/thrombotic thrombocytopenic purpura, Dengue hemorrhaigic fever, pelvic inflammatory disease, Legionella, Lyme disease, Influenza A, Epstein-Barr virus, encephalitis, inflammatory diseases and autoimmunity including Rheumatoid arthritis, osteoarthritis, systemic lupus erythematosus, inflammatory bowel disease, idiopathic pulmonary fibrosis, sarcoidosis, hypersensitivity pneumonitis, systemic vasculitis, Wegener's granulomatosis, transplants including heart, liver, lung kidney bone marrow, graft-versus-host disease, transplant rejection, sickle cell anemia, nephrotic syndrome, toxicity of agents such as OKT3, cytokine therapy, and cirrhosis.

13. The method of any one of claims 1-12, wherein the inflammatory condition is systemic inflammatory response syndrome.

14. A method for selecting a group of patients for determining the efficacy of a candidate drug known or suspected of being useful for the treatment of an inflammatory condition, the method comprising determining a genotype for one or more polymorphism sites in the plasminogen-activator-inhibitor-1 gene for each patient, wherein said genotype is indicative of the patient's ability to recover from the inflammatory condition and sorting patients based on their genotype, provided that the polymorphism site is not solely a polymorphism at a site corresponding to position 837 of SEQ ID NO.: 1.

15. The method of claim 14 further comprising, comparing patient response to the candidate drug based on genotype of the patient.

16. The method of claim 15, wherein patient response is determined by each patient's ability to recover from the inflammatory condition.

17. The method of claim 15, wherein the one or more polymorphism sites are selected from positions 5645, 7121, 7437, 8070, 8406, 9463, 9466,12219, 12580, 13889 and 14440 in SEQ ID NO: 1.

## Patentansprüche

1. Verfahren zur Erzielung einer Prognose für einen Patienten, der einen entzündlichen Zustand hat oder gefährdet ist, einen entzündlichen Zustand zu entwickeln, umfassend die Bestimmung eines Genotyps für eine oder mehrere Polymorphismusstelle(n) im Plasminogen-Aktivator-Inhibitor-1 (PAI-1)-Gen des Patienten, wobei der Genotyp ein Anzeiger für eine Fähigkeit des Patienten zur Erholung von einem entzündlichen Zustand ist, vorausgesetzt die eine oder mehreren Polymorphismusstelle(n) ist (sind) nicht nur ein Polymorphismus an einer Stelle, die Position 837 in SEQ ID Nr.: 1 entspricht.

2. Verfahren gemäß Anspruch 1, wobei die eine oder mehreren Polymorphismusstelle(n) Position 12580 der SEQ ID Nr.: 1 einschließt (einschliessen).

3. Verfahren gemäß Anspruch 1, wobei die eine oder mehreren Polymorphismusstelle(n) ausgewählt ist (sind) aus den Positionen 5645, 7121, 7437, 8070, 8406, 9463, 9466, 12219, 12580, 13889 und 14440 in SEQ ID Nr.: 1.

4. Verfahren gemäß Anspruch 1, wobei der Genotyp eine Kombination von zwei oder mehreren Polymorphismusstellen umfasst, wobei die Kombination ausgewählt ist aus der Gruppe der Positionen, die den Positionen in SEQ ID Nr.: 1 entsprechen, bestehend aus:
664 A und 2037 T;
664 A und 2362 -;
664 A und 2852 A;
664 A und 5834 A;
837 - und 2037 T;
837 - und 2362 -;
837 - und 2852 A;
837 - und 5834 A;
| | | | | | |
|---|---|---|---|---|---|
| eine von | 5878 G | und eine von | 7365 T | und eine von | 4588 T |
| | 7343 G | | 7729 + | | 5404 G |
| | 13605 A | | 7771 A | | 5686 A |
| | | | 12750 A | | 5984 A |
| | | | | | 11312 A; |
und
| | | | | | | |
|---|---|---|---|---|---|---|
| eine von | 2846 A | und 10381 T | und eine von | 7365 T | und eine von | 4588 T |
| | 6821 T | und 10381 T | | 7729 + | | 5404 G |
| | 9759 G | und 10381 T | | 7771 A | | 5686 A |
| | | | | 12750 A | | 5984 A |
| | | | | | | 11312 A |

5. Verfahren gemäß einem der Ansprüche 1 bis 4, das weiterhin den Vergleich des so bestimmten Genotyps mit bekannten Genotypen umfasst, die Anzeiger für eine Prognose für die Erholung von dem gleichen entzündlichen Zustand wie der des Patienten oder einem anderen entzündlichen Zustand sind.

6. Verfahren gemäß einem der Ansprüche 1 bis 5, wobei die Genotyp-Bestimmung an einer Nukleinsäureprobe des Patienten ausgeführt wird.

7. Verfahren gemäß einem der Ansprüche 1 bis 6, wobei die Genotyp-Bestimmung eine oder mehrere der folgenden umfasst:
(a) Restriktionsfragment-Längenanalyse;
(b) Sequenzierung;
(c) Hybridisierung;
(d) Oligonukleotid-Ligationsassay;
(e) Ligations-Rolling-Circle-Amplifizierung;
(f) 5'-Nukleaseassay;
(g) Polymerase-Proofreading-Verfahren;
(h) Allel-spezifische PCR; und
(i) Lesen von Sequenzdaten.

8. Verfahren gemäß einem der Ansprüche 1 bis 7, wobei der Genotyp des Patienten ein Anzeiger für eine verminderte Wahrscheinlichkeit der Erholung einem entzündlichen Zustand ist.

9. Verfahren gemäß Anspruch 8, wobei die Prognose ein Anzeiger für schwere kardiovaskuläre oder respiratorische Funktionsstörung in kritisch kranken Patienten ist.

10. Verfahren gemäß einem der Ansprüche 1 bis 7, wobei der Genotyp des Patienten ein Anzeiger für eine erhöhte Wahrscheinlichkeit der Erholung von einem entzündlichen Zustand ist.

11. Verfahren gemäß Anspruch 10, wobei die Prognose ein Anzeiger für eine weniger schwere kardiovaskuläre oder respiratorische Funktionsstörung in kritisch kranken Patienten ist.

12. Verfahren gemäß einem der Ansprüche 1 bis 11, wobei der entzündliche Zustand ausgewählt ist aus der Gruppe bestehend aus: Sepsis, Septikämie, Pneumonie, septischer Schock, systemisches Entzündungsreaktionssyndrom (SIRS), akutes Atemnotsyndrom (ARDS), akute Lungenverletzung, Infektion, Pankreatitis, Bakteriämie, Peritonitis, abdominaler Abszess, Entzündung, ausgelöst durch Trauma, Entzündung, ausgelöst durch Operation, chronische entzündliche Erkrankung, Ischämie, Ischämie-Reperfusionsschaden eines Organs oder Gewebes, Gewebeschädigung, ausgelöst durch Erkrankung, Gewebeschädigung, ausgelöst durch Chemotherapie oder Radiotherapie und Reaktionen auf verschluckte, eingeatmete, infundierte, injizierte oder gegebene Substanzen, Glomerulonephritis, Darminfektion, opportunistische Infektionen, und für Patienten, die einer größeren Operation oder Dialyse unterzogen werden, Patienten, die immunsupprimiert sind, Patienten auf Immunosuppressiva, Patienten mit HIV/AIDS, Patienten mit vermuteter Endokarditis, Patienten mit Fieber, Patienten mit Fieber unbekannten Ursprungs, Patienten mit cystischer Fibrose, Patienten mit Diabetis mellitus, Patienten mit chronischem Nierenversagen, Patienten mit Bronchiektase, Patienten mit chronisch obstruktiver Lungenerkrankung, chronischer Bronchitis, Emphysema oder Asthma, Patienten mit fiebriger Neutropenie, Patienten mit Meningitis, Patienten mit septischer Arthritis, Patienten mit Harnwegsinfektion, Patienten mit nekrotisierender Fasziitis, Patienten mit anderer vermuteter Gruppe A Streptococcus-Infektion, Patienten, an denen Splenektomie vorgenommen wurde, Patienten mit wiederkehrender oder vermuteter Enterococcus-Infektion, andere medizinische oder chirurgische Beschwerden, die mit erhöhtem Risiko für Infektionen verbunden sind, Gram-positive Sepsis, Gram-negative Sepsis, Kulturnegative Sepsis, fungale Sepsis, Meningokokkämie, Post-Pump-Syndrom, Cardiac-Stun-Syndrom, myokardialer Infarkt, Schlaganfall, kongestive Herzinsuffizienz, Hepatitis, Epiglotittis, E. coli 0157:H7, Malaria, Gasbrand, toxisches Schocksyndrom, myobakterielle Tuberkulose, Pneumocystis carinii-Pneumonie, Leishmaniose, hämolytisches urämisches Syndrom/thrombotische thrombocytopenische Purpura, hämorrhagisches Dengue-Fieber, entzündliche Beckenerkrankung, Legionella, Borreliose, Influenza A, Epstein-Barr-Virus, Encephalitis, entzündliche Erkrankungen und Autoimmunität einschließlich rheumatoide Arthritis, Osteoarthritis, systemischer Lupus erythematosus, entzündliche Darmerkrankung, idiopatische Lungenfibrose, Sarcoidose, Hypersensitivitätspneumonitis, systemische Vaskulitis, Wegener's Granulomatose, Transplantate einschließlich Herz, Leber, Lunge, Niere, Knochenmark, Graft-Versus-Host-Erkrankung, Transplantatabstoßung, Sichelzellenanämie, nephrotisches Syndrom, Toxizität von Agenzien wie OKT 3, Cytokintherapie und Zirrhose.

13. Verfahren gemäß einem der Ansprüche 1 bis 12, wobei der entzündliche Zustand systemisches Entzündungsreaktionssyndrom ist.

14. Verfahren zur Auswahl einer Gruppe von Patienten für die Bestimmung der Wirksamkeit eines Wirkstoffkandidaten, für den bekannt ist oder für den vermutet wird, dass er für die Behandlung eines entzündlichen Zustands nützlich ist, umfassend die Bestimmung eines Genotyps für eine oder mehrere Polymorphismusstelle(n) im Plasminogen-Aktivator-Inhibitor-1-Gen jedes Patienten, wobei der Genotyp ein Anzeiger für die Fähigkeit des Patienten, sich von dem entzündlichen Zustand zu erholen, ist, und die Klassifizierung der Patienten auf Grundlage ihrer Genotypen, vorausgesetzt dass die Polymorphismusstelle nicht nur ein Polymorphismus an der Stelle, die Position 837 in SEQ ID Nr.: 1 entspricht, ist.

15. Verfahren gemäß Anspruch 14, das weiterhin den Vergleich der Patientenreaktion auf den Wirkstoffkandidaten, basierend auf den Genotyp des Patienten, umfasst.

16. Verfahren gemäß Anspruch 15, wobei die Patientenreaktion durch die Fähigkeit jedes Patienten bestimmt wird, sich von dem entzündlichen Zustand zu erholen.

17. Verfahren gemäß Anspruch 15, wobei die eine oder die mehreren Polymorphismusstelle(n) ausgewählt ist (sind) aus den Positionen 5645, 7121, 7437, 8070, 8406, 9463, 9466, 12219, 12580, 13889 und 14440 in SEQ ID Nr.: 1.

## Revendications

1. Procédé pour obtenir un pronostic pour un patient présentant ou à risque de développer un état inflammatoire, le procédé comprenant la détermination d'un génotype pour un ou plusieurs site(s) de polymorphisme dans le gène de l'inhibiteur 1 de l'activateur du plasminogène (PAI-1) pour le patient, dans lequel ledit génotype est indicateur d'une capacité du patient à se remettre d'un état inflammatoire, à condition que le un ou plusieurs site(s) de polymorphisme ne soit(soient) pas uniquement un polymorphisme au niveau d'un site correspondant à la position 837 de SEQ ID NO: 1.

2. Procédé selon la revendication 1, dans lequel le un ou plusieurs site(s) de polymorphisme inclut(incluent) la position 12580 de SEQ ID NO: 1.

3. Procédé selon la revendication 1, dans lequel le un ou plusieurs site(s) de polymorphisme est(sont) choisi(s) parmi les positions 5645, 7121, 7437, 8070, 8406, 9463, 9466, 12219, 12580, 13889 et 14440 dans SEQ ID NO: 1.

4. Procédé selon la revendication 1, dans lequel le génotype comprend une combinaison de deux sites de polymorphisme ou plus, la combinaison étant choisie dans le groupe de positions correspondant à SEQ ID NO: 1 constitué de :
664 A et 2037 T ;
664 A et 2362 - ;
664 A et 2852 A ;
664 A et 5834 A ;
837 - et 2037 T ;
837 - et 2362 - ;
837 - et 2852 A ;
837 - et 5834 A ;
| | | | | | |
|---|---|---|---|---|---|
| une parmi | 5878 G | et une parmi | 7365 T | et une parmi | 4588 T |
| | 7343 G | | 7729 + | | 5404 G |
| | 13605 A | | 7771 A | | 5686 A |
| | | | 12750 A | | 5984 A |
| | | | | | 11312 A |
; et
| | | | | | |
|---|---|---|---|---|---|
| une parmi | 2846 A et 10381 T | et une parmi | 7365 T | et une parmi | 4588 T |
| | 6821 T et 10381 T | | 7729 + | | 5404 G |
| | 9759 G et 10381 T | | 7771 A | | 5686 A |
| | | | 12750 A | | 5984 A |
| | | | | | 11312 A. |

5. Procédé selon l'une quelconque des revendications 1 à 4, comprenant en outre la comparaison du génotype ainsi déterminé avec des génotypes connus, qui sont indicateurs d'un pronostic de rétablissement du même état inflammatoire que celui du patient ou d'un autre état inflammatoire.

6. Procédé selon les revendications 1 à 5, dans lequel ladite détermination de génotype est réalisée sur un échantillon d'acide nucléique provenant du patient.

7. Procédé selon l'une quelconque des revendications 1 à 6, dans lequel ladite détermination de génotype comprend un ou plusieurs parmi :
(a) une analyse de longueur d'un fragment de restriction ;
(b) un séquençage ;
(c) une hybridation ;
(d) un essai de ligature d'oligonucléotide ;
(e) une amplification par cercle tournant ligature ;
(f) un essai 5' nucléase ;
(g) des procédés de correction d'épreuve par polymérase ;
(h) une PCR spécifique d'un allèle ; et
(i) une donnée de séquence de lecture.

8. Procédé selon l'une quelconque des revendications 1 à 7, dans lequel le génotype du patient est indicateur d'une probabilité réduite de rétablissement d'un état inflammatoire.

9. Procédé selon la revendication 8, dans lequel le pronostic est indicateur d'une dysfonction cardiovasculaire ou respiratoire grave chez des patients gravement malades.

10. Procédé selon l'une quelconque des revendications 1 à 7, dans lequel le génotype du patient est indicateur d'une probabilité accrue de rétablissement d'un état inflammatoire.

11. Procédé selon la revendication 10, dans lequel le pronostic est indicateur d'une dysfonction cardiovasculaire ou respiratoire moins grave chez des patients gravement malades.

12. Procédé selon l'une quelconque des revendications 1 à 11, dans lequel l'état inflammatoire est choisi dans le groupe constitué de : une sepsie, une septicémie, une pneumonie, un choc septique, un syndrome de réponse inflammatoire systémique (SRIS), un syndrome de détresse respiratoire aiguë (SDRA), une lésion pulmonaire aiguë, une infection, une pancréatite, une bactériémie, une péritonite, un abcès abdominal, une inflammation due à un trauma, une inflammation due à une opération chirurgicale, une maladie inflammatoire chronique, une ischémie, une lésion par ischémie-reperfusion d'un organe ou d'un tissu, une lésion tissulaire due à une maladie, une lésion tissulaire due à une chimiothérapie ou une radiothérapie, et des réactions à des substances ingérées, inhalées, perfusées, injectées, ou délivrées, une glomérulonéphrite, une infection intestinale, des infections opportunistes, et pour les patients subissant une opération chirurgicale ou une dialyse lourde, les patients qui sont immunodéprimés, les patients sous agents immunodépresseurs, les patients avec un VIH/SIDA, les patients avec une suspicion d'endocardite, les patients atteints de fièvre, les patients atteints d'une fièvre d'origine inconnue, les patients atteints de fibrose kystique, les patients atteints de diabète sucré, les patients atteints d'insuffisance rénale chronique, les patients atteints de bronchiectasie, les patients atteints d'une maladie pulmonaire obstructive chronique, une bronchite chronique, un emphysème, ou un asthme, les patients atteints de neutropénie fébrile, les patients atteints de méningite, les patients atteints d'une arthrite septique, les patients présentant une infection du tractus urinaire, les patients atteints de fasciite nécrosante, les patients atteints d'une autre infection soupçonnée d'être à streptocoque du groupe A, les patients qui ont subi une splénectomie, les patients atteints d'une infection à entérocoque récurrente ou suspectée, d'autres états médicaux et chirurgicaux associés à un risque accru d'infection, une sepsie à Gram positif, une sepsie à Gram négatif, une sepsie avec culture négative, une sepsie fongique, une méningococcémie, un syndrome post-pompe, un syndrome de paralysie cardiaque, un infarctus du myocarde, un accident vasculaire cérébral, une insuffisance cardiaque congestive, une hépatite, une épiglottite, *E*. *coli* 0157:H7, un paludisme, une gangrène gazeuse, un syndrome de choc toxique, une tuberculose mycobactérienne, une pneumonie à Pneumocystis carinii, une leishmaniose, un syndrome urémique hémolytique/purpura thrombocytopénique thrombotique, une dengue hémorragique, une maladie inflammatoire pelvienne, une légionelle, la maladie de Lyme, la grippe A, le virus d'Epstein-Barr, une encéphalite, les maladies inflammatoires et l'auto-immunité incluant l'arthrite rhumatoïde, une ostéoarthrite, un lupus érythémateux systémique, une maladie intestinale inflammatoire, une fibrose pulmonaire idiopathique, une sarcoïdose, une pneumopathie d'hypersensibilité, une vascularite systémique, une granulomatose de Wegener, les transplants incluant le coeur, le foie, le poumon, le rein, la moelle osseuse, la maladie du greffon contre l'hôte, le rejet de transplant, une anémie falciforme, un syndrome néphrotique, la toxicité d'agents tels que l'OKT3, une thérapie par cytokine, et une cirrhose.

13. Procédé selon l'une quelconque des revendications 1 à 12, dans lequel l'état inflammatoire est un syndrome de réponse inflammatoire systémique.

14. Procédé pour sélectionner un groupe de patients afin de déterminer l'efficacité d'un médicament candidat connu ou soupçonné d'être utile pour le traitement d'un état inflammatoire, le procédé comprenant la détermination d'un génotype pour un ou plusieurs site(s) de polymorphisme dans le gène de l'inhibiteur 1 de l'activateur du plasminogène pour chaque patient, dans lequel ledit génotype est indicateur de la capacité du patient à se remettre de l'état inflammatoire et le tri des patients sur la base de leur génotype, à condition que le site de polymorphisme ne soit pas uniquement un polymorphisme au niveau d'un site correspondant à la position 837 de SEQ ID NO: 1.

15. Procédé selon la revendication 14 comprenant en outre, la comparaison de la réponse du patient au médicament candidat sur la base du génotype du patient.

16. Procédé selon la revendication 15, dans lequel la réponse du patient est déterminée par la capacité de chaque patient à se remettre de l'état inflammatoire.

17. Procédé selon la revendication 15, dans lequel le un ou plusieurs site(s) de polymorphisme est(sont) choisi(s) parmi les positions 5645, 7121, 7437, 8070, 8406, 9463, 9466, 12219, 12580, 13889 et 14440 dans SEQ ID NO: 1.


### SEQUENCE LISTING

<110> THE UNIVERSITY OF BRITISH COLUMBIA
   RUSSELL, James A.
   WALLEY, Keith R.
<120> PLASMINOGEN ACTIVATOR INHIBITOR-1 (PAI-1) HAPLOTYPES USEFUL AS
   INDICATORS OF PATIENT OUTCOME
<130> 80021-638
<140> NOT YET ASSIGNED
   <141> 2004-03-19
<150> US 60/455,550
   <151> 2003-03-19
<160> 11
<170> PatentIn version 3.2
<210> 1
   <211> 14544
   <212> DNA
   <213> Homo sapiens
<400> 1
   cgtactggtc catagcctgt taggaaccag gctgcataac aggaggtgag tggcaggtga    60
   gtgaaatttc atctgtagtt acagccactc ctcatcactc gcattaccac cagagctcca   120
   ctccctgtca gatcagcggc ggcattagat tctcatagga gctcgaaccc tattctaaac   180
   tgttcatgtg agggatctag gttgcaagct ccctatgaga atctaatgcc tgatgatctg   240
   tcacggtctc ccatcacccc tagatgggac catctagttg caggaaaaca agctcagggc   300
   tcccactgat tctacacgat ggtgaattgt ggaattattt cattatatat attacaatgt   360
   aataataata gaaataaagc acacaataaa tgtaatgtgc ttgaatcatc ccgaaaccat   420
   cccaccctgg tctgtgaaaa aattgtcttc catgaaacca gtccctggtg ccaaaaacgt   480
   tgaggaccac tgctccacag aatctatcgg tcactcttcc tcccctcacc cccttgccct   540
   aaaagcacac cctgcaaacc tgccatgaat tgacactctg tttctatccc ttttcccctt   600
   gtgtctgtgt ctggaggaag aggataaagg acaagctgcc ccaagtccta gcgggcagct   660
   cgaggaagtg aaacttacac gttggtctcc tgtttcctta ccaagctttt accatggtaa   720
   cccctggtcc cgttcagcca ccaccacccc acccagcaca cctccaacct cagccagaca   780
   aggttgttga cacaagagag ccctcagggg cacagagaga gtctggacac gtgggggagt   840
   cagccgtgta tcatcggagg cggccgggca catggcaggg atgagggaaa gaccaagagt   900
   cctctgttgg gcccaagtcc tagacagaca aaacctagac aatcacgtgg ctggctgcat   960
   gccctgtggc tgttgggctg ggcccaggag gagggagggg cgctctttcc tggaggtggt   1020
   ccagagcacc gggtggacag ccctggggga aaacttccac gttttgatgg aggttatctt   1080
   tgataactcc acagtgacct ggttcgccaa aggaaaagca ggcaacgtga gctgtttttt  1140
   ttttctccaa gctgaacact aggggtccta ggctttttgg gtcacccggc atggcagaca  1200
   gtcaacctgg caggacatcc gggagagaca gacacaggca gagggcagaa aggtcaaggg  1260
   aggttctcag gccaaggcta ttggggtttg ctcaattgtt cctgaatgct cttacacacg  1320
   tacacacaca gagcagcaca cacacacaca cacacacatg cctcagcaag tcccagagag  1380
   ggaggtgtcg agggggaccc gctggctgtt cagacggact cccagagcca gtgagtgggt  1440
   ggggctggaa catgagttca tctatttcct gcccacatct ggtataaaag gaggcagtgg  1500
   cccacagagg agcacagctg tgtttggctg cagggccaag agcgctgtca agaagaccca  1560
   cacgcccccc tccagcagct gaattcctgc agctcagcag ccgccgccag agcaggacga  1620
   accgccaatc gcaaggcacc tctgagaact tcaggtagga gaaaagcaaa ctccctccaa  1680
   cctcttactt cgggcttaag gcagagaact cgcctcccca gaatctcctc cctccatgat  1740
   cccccgctat tcctctattt tcttttcctc ggacctgcag ccttgggtcg accctgccct  1800
   aggggtgact gcaggagagc agggaggatg gtcaggcgtc accaacaacc ccatcaccca  1860
   gtaacaagaa ccttgactct ctcagtccct ctgcatcaag acacttaccc atttcccacc  1920
   tcatgcctgc taacttgaat gaaacaatcg ctgggaaagc attaagagaa ttaaggctgg  1980
   gcactgtggc tcatgcctgt aatcccagca ctttgtgagg ctgaggcagg cagataactt  2040
   gagcccagga gtttgagacc agcctgggca acatggcaaa accctgctct cccaaaaaaa   2100
   tacaaaaatt agctgggcgt gctggtgtgc ctgtattccc agctacttgg gaggctgagg   2160
   tgggaggatt gcttcagctg gggaggcgga ggctgcaggg agccaagact gagccattgc   2220
   acccagcctg ggtgacagag caagaccctg tctctaaaaa tgaatgaaag gaaggaagaa   2280
   agagagagaa agagagagag gaaagaagga aggaagtaaa gaagaaagaa agaaagaaag   2340
   aggaaagagg aagaaagaaa gaaaagaaag aaaagaaaag aaagcaaatt taaagcttat    2400
   gcaaatcaaa gatgttgtga taattgataa ttgagtctgg gctaaattcc ccctgggctg    2460
   caaaggcaga gagtggtaat gacttctcac ctgcttttct tctaaggctt ttttacggga   2520
   cacagaggga agggagatgg actggattcc aagattccca cagggcaaga tgggcgaaga   2580
   ctccctgcca ctgcccgggg ataagtcagt ctgagtgaga cggagtggga tgggcttaga   2640
   acctgaacat gtcatggtct cttcctgcac cttgccctag tgttcactta ccacctgctt   2700
   gcaggaaaca agaagagcag ggcccacagc tggccagctc ccctcccctc ccgcctgtct   2760
   tccagaacga ttccttcacc agccctcttt ccattgctct aggatgcaga tgtctccagc   2820
   cctcacctgc ctagtcctgg gcctggccct tgtctttggt gaagggtctg ctgtgcacca   2880
   tcccccatcc tacgtggccc acctggcctc agacttcggg gtgagggtgt ttcagcaggt  2940
   ggcgcaggcc tccaaggacc gcaacgtggt tttctcaccc tatggggtgg cctcggtgtt   3000
   ggccatgctc cagctgacaa caggaggaga aacccagcag cagattcaag cagctatggg   3060
   attcaagatt gatggtgagc cacgggacac caggggaggt gggtggcatg cagaacagac   3120
   ctaccagaag ccaaggaaag gctggctctg gcttagccga gccaagcccc atacagctgt   3180
   gctgcagggg ccaccccatc ttcttcccac tacactccaa gtcactggac ccttgaatct   3240
   ccaagggtgt ctgaccagta gatttaccgc ttattcacca ccgtgtgatc ttaacctcgt   3300
   taagtttgcc catctacaaa atgaggatta tttgctgtcc taaagaattc atgagccggg   3360
   cgcggtggct caaacgcctg taatcccagc actttgggag gccaaggcgg gcggatcatg   3420
   aggtcaggag atcaagacca tcctggctaa cacagtgaaa ctccatctct actaaaaata   3480
   caaaaaaaat tagccaggcg tggtggcagg cgcctgtagt cccagctact cgggaggctg   3540
   aggcaggaga atggcatgaa cccaggaggc agagcttgca gtgagctgag atcgtgccac   3600
   tgcactccag cctgggcgac agagagagac tccgtctcaa aaaaaaaaat taaaagaatt    3660
   catggaatta cacttgtgaa atacttagca tagccatcac tataggaaaa aaatctaagg   3720
   ccaggcacag tgcctcatgc ctgtaatctc agcactttcg gagtttgagg caggaggatc   3780
   acccaaggct aggagttcaa ggccagcctg ggcaatacgg tgaaaccccg tctctaataa   3840
   aaatataaaa attagtctga tgaggtggtg cacctgtaat cccagctact caggaagctg   3900
   agacacaaga atcactttaa cccgggaggt ggaggtggca gtgagctgag atcacaccat   3960
   tgcactccag cctgggtgac agagtgagac ctgtcaaaaa aaagaaaaga aagagagaga   4020
   gagagagaag agagagaaag aaagaagaag aaagaaagaa agagagagag agaaagaaag   4080
   aaagaaacaa agaaacaaag aaagaaagga aaagaaaaaa aaaactaagg ccaggcaagg   4140
   tggcttatga ctgtaatttc agcactttgg aagattgagg caggaggatc acttgaggcc   4200
   agaagttcga gacaagactg agcaacaggg agacccctgc ctctacaaaa aaatttacaa   4260
   attagccaga tgtggtgaca catacctgta gtcccaacta ctcaggaggc tgaggtggga   4320
   ggatggcttg agcccaggag ctggaggctg cagtgagcta tgattgtacc actgcacttc   4380
   agcctgggca acaaagggaa gccctgtctg aaaaaaaaaa aaaaagaaaa agaagaagaa   4440
   agaaaatatt tagggttcat ccaggaggca gaggttgcag taagctgaca tcgcgccatt   4500
   gcactccagc ctgggagaca agagcaaaac tccaactcaa aaaaaaaaaa aaaaaaaaaa   4560
   caggaagaaa atatttaggg ttcataactt aagaacagag aaaaatattc tagcccaaag   4620
   aaagggttgg gatctgagac ttttgaagaa aggaaggaga tacagaaaag agatttcatc   4680
   ctggaatgaa atctccctcc agagagccct gggaaagcac ggtagccccc atccatcaga   4740
   gtggagcccc ttgtggggga agtgggctcg gctgggaacc ctcaattcag cataagcctc   4800
   acatgtcctc tcctctctgt cccggtgcag acaagggcat ggcccccgcc ctccggcatc    4860
   tgtacaagga gctcatgggg ccatggaaca aggatgagat cagcaccaca gacgcgatct   4920
   tcgtccagcg ggatctgaag ctggtccagg gcttcatgcc ccacttcttc aggctgttcc   4980
   ggagcacggt caagcaagtg gacttttcag aggtggagag agccagattc atcatcaatg   5040
   actgggtgaa gacacacaca aaaggtgagc aggcagggaa aggaaaccca tttcctgggc   5100
   ctcaagagaa agggaatttg gaaataaatc cacatatccc agttgggtgc agtagttcac   5160
   acctgtaatc ccagcccaac actttgggag gtctaggcga gaggaaggct tgaggcctgg   5220
   agtttgagac cagcctggcc aacataacaa gacctcatct cttcaaaaaa tttaaaaacc   5280
   agccgggcat ggtggtgcac acctgtagtc ccagctactt gggaggctga ggtgggagga   5340
   tcacttgagt ccagcagttc aaggctgcag tgagctatgt ttgcaccacc acactccagc   5400
   ctgagtcaca gaacaagacc tcatctctaa aaaacaaaca aaaaccaaat ccacatatcc   5460
   taaaaaatgc tccttttcag cattctcttc tctatggaca aagggctgga tgctttaaga  5520
   accaaatctt aggctgggca cggtggctca cgcctctaat cctagcactt tgagaggcca   5580
   aggcgggcag attgcctgag cacaggagtt cgagaccagc ctggccaaca tggtgaaacc   5640
   ctgtctctgt caaaaataca aaaaattagc caggtgtgtt ggcgcgtgcc tataatccca   5700
   gctgctcggg aggatgaggt tcaaagaatc acttgaaccc gggaggcaga ggctgcagtg 5760
   agctgagatc atgccactgc actccagcct gggtgacaga gcaagacttt gtctccaaaa 5820
   aaaggaacta gacgggttca tttaaacccc tgactgcagc cctttgacat acatccaatt 5880
   gaggactggg gactccggga aacatctaaa aggcttaaaa actttgtcta acttcagccg 5940
   ggcatggtgg ctcacacctg taatcccagc actttgggag gctgaggcag gtggatcaca 6000
   aggtcaggag tttgagacga gcctgaccaa catggtgaaa ccccgtctct actaaaaata 6060
   caaaaattag ccaggcatgg tggcaggcgc ctgtaatccc agctattcgg gaggctgagg 6120
   caggagaatt gcttgaaccc cggagacaga ggttgcagcg agccgagatc gcgccactgc 6180
   actccagcct ggcaatagag tgagactcca tctcaaaaca acaacaacaa caacaacaac 6240
   aacaaaatcg tctaacttcc tgatcttcct gatcattgat tttcccatag gtatgatcag 6300
   caacttgctt gggaaaggag ccgtggacca gctgacacgg ctggtgctgg tgaatgccct 6360
   ctacttcaac ggccagtgga agactccctt ccccgactcc agcacccacc gccgcctctt 6420
   ccacaaatca gacggcagca ctgtctctgt gcccatgatg gctcagacca acaagttcaa 6480
   ctatagtaag tccaagagcc ccttccccac agcccacagc aactgcatct cattcctggg 6540
   gtctcccaag gaatacccaa aatgtcaccc tctgagggag gaagaccaca gggaatgctc 6600
   ccctttaagg gaggagagac cctagaatat actccagctt tgacaaagat ttcccaagca 6660
   ggagacatca ggataatggg aacagaagac aggaggttta tcccatgaag gatgaagaag 6720
   ctgaaatcca gagattccct cagggccaca tttgtccacc tgactccagg gtctcatctt 6780
   cgtgtgttgc tagtgtgatt acctggggat gagaaatcct gctgggggag ttgaggttaa 6840
   gaggatgagg actccaggtg ctgtggctca cgcctgtaat cccagcactt tgggaggcca 6900
   aggcaggtgg atcaggagtt tgaggtcagg agtttgagac cagcctggcc aacatggtga 6960
   aaccctgtct ctactaaaaa tgcaaaaatt agccaggtgt ggtggcaggc gcctgtaatc 7020
   ccagctactc gggaggctga ggcaggagaa tcacttgagc ccgggaggtg gaggttgcag 7080
   tgagccgaac gaaattgagc cacttcaccc cagcctgggc aaaagagtga aattccattc 7140
   aaaaaaaaaa aaaaaaaaaa aaggatgagg actgggatga actggtggct gggtgtgggg 7200
   aaaatggaag tgaaggaagg ccaaaagaga cagagaaggc ctggcgcggc gactcacgcc 7260
   tataatccca gcactttggg aggctgagaa gggggattgc ttgaggccag aagttgaata 7320
   ccagtctggg cagcatagca agaccctgcc tctacaaaaa aaaaattttt tttaattagc 7380
   caggcttggt gacatgcatc tgtagtctac tcaagaagct gaggtgaggc caggcacggt 7440
   ggctcacgcc tgtattccca gcactttggg aggtcaaggc gggtggatga cctgaggtca 7500
   ggagttcaag accagcctgg ccaacatggt gaaaccccat ctgtataaaa atacaaaaat 7560
   tagctgggca tgatagcagg tgcctgtaat tccagctact caggaggctg aggtgggaga 7620
   atctattgaa cccgggaggg ggaggttgca gtgagccgag atcatgccat tgcactccag 7680
   cctgggcgac agagtgagac tccttctcaa aacaaacaaa caaacaaaca aacaaaatac 7740
   agaagctgag gcgggaggaa catttgaacc ggattcggag gctgcagtga gctatgattg 7800
   caccactgcg ctccagtctg tgtgacagtg agaccctgtc tcttacacac acacacacac 7860
   acacacacac acatgcacac acacagagag agagaaatta gaagatactg aattggcaga 7920
   agagaaggga aatagaaatt aaaatactga ataggggagc agtgaacagg ggatacccaa 7980
   aagccaagag cgagagagag cctggcttcc agaaatagtg gagaagccag gagaactagg 8040
   tgaaaaccca gtgctgggtt gccatcagcg agagctggag ccatttccaa cgaaccatct 8100
   tgtcgtcttc acagctgagt tcaccacgcc cgatggccat tactacgaca tcctggaact 8160
   gccctaccac ggggacaccc tcagcatgtt cattgctgcc ccttatgaaa aagaggtgcc 8220
   tctctctgcc ctcaccaaca ttctgagtgc ccagctcatc agccactgga aaggcaacat 8280
   gaccaggctg ccccgcctcc tggttctgcc caagtaagcc accccgctat ctccccgacc 8340
   taccaacccc tctctcctgg ctccctaaag tcaccgcccc caggttgaat ttcccagatc 8400
   tgtgatgctt gcaggacatg catgtgtggg aggctgatgg gaaactgtgg cctgggtttg 8460
   attatgagtc ttgcaatcat ccctccccct gtttctgctg gagggcaggg gacagctctt 8520
   cctgaccaca cccccacatt gactatcccc agaataccca gcaaaagccc ccaaaaggag 8580
   agtcagagaa atgagggagg tgggggccca atcagtccac atctacttag ggtcgcccca 8640
   tcagcacttc catccccaac cctttcaagt caacatccaa acaaaagaaa tcacttccaa 8700
   ggacggagca gctcaaagcg cagcttctag ctggggttcc aagaaagcag atttttcgaa 8760
   atccttctgc agaaggaagc aaagagattt tttgaaatct ttctgcagaa ggagaaggct 8820
   ggagctgggg aactccagaa ttatagggaa gcctcccacc acgctcatcc caaatttccg 8880
   gatgctataa tgccaggctt ggggaaagag gagaatttag ttggttagct ggtgcgtgct 8940
   ctcacttgca tcctctctct tcctcttttt tttttttctc ctctctctct ggctcataaa 9000
   aatggaggta attagttgtg ccctggtgag aagcagagag tgcacaaagg ccccctgctt 9060
   gagtcctctt cagggttagc tctcagaaac acaatctgca gaacagattt ttgttccaac 9120
   atccttgcag gagaatttgc ccttagcttc ccccacccca gccaggctga ataaaattat 9180
   gctgaaacta ctgtcttatt tgaggaaagt aattagtcat aggtgggagg gggtggggag 9240
   attgcagaag aatgttcatg aatattagga ttttcagctc taagggggga ctttgtaaac 9300
   agctttagaa gaagaaccag gccggctggg tgtggtggct catgcctgta atctcagcat 9360
   ttggggaggc caaggcgggc ggatcacttg aggtcaggag tttgagacca gcctggccaa 9420
   catggtgaaa ccctgtctct attaaaaata caaaaattag ccagccgtgg tagcgagcgc 9480
   ctatgatccc agctactccg gaggctgagg ccagagaatc acatgaacct gggaggtgga 9540
   ggctgcagtg agccgagatc acgccactgc actccagcct gggggacaga gcaagaatct 9600
   gtttcaaaaa aaaaaaaaga aaaataggaa ggaaggaagg aaaggaaagg aaagaagaga 9660
   gagagaaaga aagagagaga gagagagaga aagaaagaaa gaaagaaaga aagaaagaaa 9720
   gaaagaaaga aagaaagaaa gaaaaagaaa ggaaagaaag aacgaacgaa ccaggcctcc 9780
   ctctccaacc ttcacctccg tccctattct ggccacttga ttcgggggac acctggtagg 9840
   ggatggggaa aggtgggagc tgccagccag aggggacccc ggcttgagca gcctcttgct 9900
   gctatctgca ggttctccct ggagactgaa gtcgacctca ggaagcccct agagaacctg 9960
   ggaatgaccg acatgttcag acagtttcag gctgacttca cgagtctttc aggtaagaag 10020
   actttccttt gcattttctc accccagtgg actgcggggg cccctaagag gaaaaaggaa 10080
   cctctccttg agagcggcag ctgatctaat cctgtatcca catctgtttc agaccaagag 10140
   cctctccacg tcgcgcaggc gctgcagaaa gtgaagatcg aggtgaacga gagtggcacg 10200
   gtggcctcct catccacagg tgagtctggc tcaggtgagg ctccacgggt gtcgcctcca 10260
   tcgcccttca ggataactgg tccccagacc cggaaaggac cccgcagccc tctcggcaca 10320
   gagcagctct gtctgtgctc agccatcacc cactccccac ctgtttctca gcctggaaaa 10380
   cgggcttggg accatggaac cctgtttcct cgcctgatgg ctcctaagtt ccctgactgt 10440
   gaaaaggcct cctaaagaaa aacccaagtt gttcccacag tgggaagtaa acttaagaaa 10500
   catgcttatc aggctgggca tggtggctcc cacctgtaat cccagcgctt tgggggacca 10560
   aggcaggtgg atcacttgag gtcaggaatt cgagaccagc ctgggcaaca tggcaaaacc 10620
   ctatctctac taaaaataca aaaattaggc aggcgtggtg gcatgtgcct gtagtcccag 10680
   ctacttggga ggctgaggca ggagaatcac ttgaatccag gaggcagagg ttgcagtgag 10740
   ccgagatcac gctgctgcac tccagcctgg gcaatagagc atgactctga agaaaagaaa 10800
   gaaagaaaga gagagagaga gaaaagaaag aaagaaagaa agaaagaaag aaagaaagaa 10860
   agaaagaaag aaagaaagaa agagaaagaa agagaagaaa agaaaagaaa gagcttatca 10920
   ataagccctt aaaggattta gataaatgtg tgtaagggaa gagctgatcc attgctacca 10980
   agctcctgga ggaaaccagg tctcagagga tgtccctaaa cttttaaggt tcatattcag 11040
   gaaaacaaac aacttccagc tgggcttagt ggctcacacc tgtaatccca gcactttggg 11100
   aggccgaggc aggaggatcg cttgagccca ggaatttgag accagcctgg gcaatataat 11160
   gagactgtgc tctacaaaaa ttagaaaaaa attagccagg catggtggca tgcacctgta 11220
   gccccagtta cttgggagac tgaggtggga ggatcacttg agcccatgag ttcaaggctg 11280
   cagtgagcca tgaaggtgcc actgcactcc cgcctgggcg acagagggag accctgtctc 11340
   taagaaaaac ggcgggggtg ggggtggtgc cagtgccagc atccctctgt tctaagacat 11400
   tgtcccttct cttgcagctg tcatagtctc agcccgcatg gcccccgagg agatcatcat 11460
   ggacagaccc ttcctctttg tggtccggca caaccccaca ggtgagcctg gaacccatca 11520
   cgttccacat cctcccaccc attctttctc tcaggaacta gtcccgacag atgcagacat 11580
   ccctctatcc ctgagagggc tctgggcagg gaaccataac cctaccctgc ttcctgtccc 11640
   aagaggaggc taccttctat cacccacaga cagtgccggg tccccgctct gtgactcagg 11700
   cagctgcgac tccagacagc tcactcatct gcctagatct cagtccttcc acccacatcc 11760
   agcctgatga gctgtcccac tccttctgct tctcaacccc catggttctt ccaccctcag 11820
   gaacagtcct tttcatgggc caagtgatgg aaccctgacc ctggggaaag acgccttcat 11880
   ctgggacaaa actggagatg catcgggaaa gaagaaactc cgaagaaaag aattttagtg 11940
   ttaatgactc tttctgaagg aagagaagac atttgccttt tgttaaaaga tggtaaacca 12000
   gatctgtctc caagaccttg gcctctcctt ggaggacctt taggtcaaac tccctagtct 12060
   ccacctgaga ccctgggaga gaagtttgaa gcacaactcc cttaaggtct ccaaaccaga 12120
   cggtgacgcc tgcgggacca tctggggcac ctgcttccac ccgtctctct gcccactcgg 12180
   gtctgcagac ctggttccca ctgaggccct ttgcaggatg gaactacggg gcttacagga 12240
   gcttttgtgt gcctggtaga aactatttct gttccagtca cattgccatc actcttgtac 12300
   tgcctgccac cgcggaggag gctggtgaca ggccaaaggc cagtggaaga aacacccttt 12360
   catctcagag tccactgtgg cactggccac ccctccccag tacaggggtg ctgcaggtgg 12420
   cagagtgaat gtcccccatc atgtggccca actctcctgg cctggccatc tccctcccca 12480
   gaaacagtgt gcatgggtta ttttggagtg taggtgactt gtttactcat tgaagcagat 12540
   ttctgcttcc ttttattttt ataggaatag aggaagaaat gtcagatgcg tgcccagctc 12600
   ttcacccccc aatctcttgg tggggagggg tgtacctaaa tatttatcat atccttgccc 12660
   ttgagtgctt gttagagaga aagagaacta ctaaggaaaa taatattatt taaactcgct 12720
   cctagtgttt ctttgtggtc tgtgtcaccg tatctcagga agtccagcca cttgactggc 12780
   acacacccct ccggacatcc agcgtgacgg agcccacact gccaccttgt ggccgcctga 12840
   gaccctcgcg ccccccgcgc cccccgcgcc cctctttttc cccttgatgg aaattgacca 12900
   tacaatttca tcctccttca ggggatcaaa aggacggagt ggggggacag agactcagat 12960
   gaggacagag tggtttccaa tgtgttcaat agatttagga gcagaaatgc aaggggctgc 13020
   atgacctacc aggacagaac tttccccaat tacagggtga ctcacagccg cattggtgac 13080
   tcacttcaat gtgtcatttc cggctgctgt gtgtgagcag tggacacgtg aggggggggg 13140
   tgggtgagag agacaggcag ctcggattca actaccttag ataatatttc tgaaaaccta 13200
   ccagccagag ggtagggcac aaagatggat gtaatgcact ttgggaggcc aaggcgggag 13260
   gattgcttga gcccaggagt tcaagaccag cctgggcaac ataccaagac ccccgtctct 13320
   ttaaaaatat atatatttta aatatactta aatatatatt tctaatatct ttaaatatat 13380
   atatatattt taaagaccaa tttatgggag aattgcacac agatgtgaaa tgaatgtaat 13440
   ctaatagaag cctaatcagc ccaccatgtt ctccactgaa aaatcctctt tctttggggt 13500
   ttttctttct ttcttttttg attttgcact ggacggtgac gtcagccatg tacaggatcc 13560
   acaggggtgg tgtcaaatgc tattgaaatt gtgttgaatt gtatgctttt tcacttttga 13620
   taaataaaca tgtaaaaatg tttcaaaaaa ataataaaat aaataaatac gaagaatatg 13680
   tcaggacagt cactgccttc accttctcca tttcacaccg gtggtacaag aaatcagaag 13740
   cctaggccag gtgtggtggt tcatgcctgt aatcccagca ctttgggaag ccgaggtggg 13800
   tggatcacct aaggtcagga gtttgagacc agcctggaca acatggtgaa accccgtctc 13860
   tactaaaaat acaaaaatta gccgggcgtg gtggctggcg cctgtaatcc cagctactcg 13920
   ggaggctgag gcaggagaat cacttgaagc caggaggcag aggttgcagt gagctgagat 13980
   tgcaccactg aactccaggc tgggtggcag agcgagactc cctctcaaaa aacaacaact 14040
   acaaagacaa caacaaaccc agaatcaaaa tcctgttggt ccatagacct catgggtgga 14100
   agagaccttc ctacatccag gttggcccaa catgggggag tccatgaaat ggtcacctca 14160
   gctctgccac aagccccaag gataagttga ttctgcccct gggaatcatc ctcaaaaagg 14220
   aaaaaaatgt tcccctgcca taaactttcc acttatgcag atgggcctgc tcgtaagtca 14280
   ctgtcactgt gggttcccaa ctctgttcat gacacttcct tccagcacca aatgcttccc 14340
   acccctctac tcccactccc cattcttcaa acccagctca agttccagtt cctccaccta 14400
   ggacttccca tggatccagc caatatcact ctcaggtccg gcgcagtggc ccacgcctgt 14460
   aatctcagca ctttgggagg ccggggcagg aagattgctt gaggccagga gtttcagacc 14520
   agcctggaca acatagtgag actc 14544
<210> 2
   <211> 20
   <212> DNA
   <213> Homo sapiens
<400> 2
   aggaagaaat gtcagatgcg 20
<210> 3
   <211> 20
   <212> DNA
   <213> Homo sapiens
<400> 3
   aggaatagag gaagaaatgt 20
<210> 4
   <211> 20
   <212> DNA
   <213> Homo sapiens
<400> 4
   tgtcagatgc gtgcccagct 20
<210> 5
   <211> 30
   <212> DNA
   <213> Homo sapiens
<400> 5
   aggaagaaat gtcagatgcg tgcccagctc 30
<210> 6
   <211> 20
   <212> DNA
   <213> Homo sapiens
<400> 6
   ataggaatag aggaagaaat 20
<210> 7
   <211> 30
   <212> DNA
   <213> Homo sapiens
<400> 7
   ttttattttt ataggaatag aggaagaaat 30
<210> 8
   <211> 40
   <212> DNA
   <213> Homo sapiens
<400> 8
   ttctgcttcc ttttattttt ataggaatag aggaagaaat 40
<210> 9
   <211> 60
   <212> DNA
   <213> Homo sapiens
<400> 9
   ttctgcttcc ttttattttt ataggaatag aggaagaaat gtcagatgcg tgcccagctc 60
<210> 10
   <211> 16
   <212> DNA
   <213> Homo sapiens
<400> 10
   gaagaaatgt cagatg 16
<210> 11
   <211> 12
   <212> DNA
   <213> Homo sapiens
<400> 11
   aagaaatgtc ag 12